# EUROPEAN PATENT SPECIFICATION

(11) **EP 3 580 350 B1**
(45) Date of publication and mention of the grant of the patent: **18.11.2020**
(21) Application number: 18706946.3
(22) Date of filing: 13.02.2018
(51) Int. Cl.: C12Q 1/68, C12N 15/10, C12P 19/34

(54) **POLYMERASE ENZYME FROM PYROCOCCUS FURIOSUS**
POLYMERASEENZYM AUS PYROCOCCUS FURIOSUS
ENZYME POLYMÉRASE DE PYROCOCCUS FURIOSUS

(30) Priority: 13.02.2017 US 201762458397 P; 10.03.2017 EP 17160396
(43) Date of publication of application: 18.12.2019
(73) Proprietor: Qiagen Sciences, LLC, Waltham, MA 02451 (US)
(72) Inventor: OLEJNIK, Jerzy, Brookline, MA 02445 (US); DELUCIA, Angela, Cambridge, MA 02138 (US); CHEN, Cheng-yao, Eugene, OR 97405 (US); HELLER, Ryan Charles, Amesbury, MA 01913 (US); SCHOENFELD, Thomas William, Topsfield, MA 01983 (US)
(74) Representative: CH Kilger Anwaltspartnerschaft mbB
(86) International application number: PCT/US2018/017998
(87) International publication number: WO 2018/148724

(56) References cited:
- WO-A1-2014/142921
- US-A1- 2015 376 582
- AREZI BAHRAM ET AL: "Efficient and high fidelity incorporation of dye-terminators by a novel archaeal DNA polymerase mutant", JOURNAL OF MOLECULAR BIOLOGY, ACADEMIC PRESS, UNITED KINGDOM, vol. 322, no. 4, 27 September 2002 (2002-09-27), pages 719-729, XP002560782, ISSN: 0022-2836, DOI: 10.1016/S0022-2836(02)00843-4 [retrieved on 2002-09-17]
- GARDNER A F ET AL: "Acyclic and dideoxy terminator preferences denote divergent sugar recognition by archaeon and Taq DNA polymerases", NUCLEIC ACIDS RESEARCH, INFORMATION RETRIEVAL LTD, vol. 30, no. 2, 15 January 2002 (2002-01-15), pages 605-613, XP002309458, ISSN: 0305-1048
- EVANS STEVEN J ET AL: "Improving dideoxynucleotide-triphosphate utilisation by the hyper-thermophilic DNA polymerase from the archaeon Pyrococcus furiosus", NUCLEIC ACIDS RESEARCH, INFORMATION RETRIEVAL LTD, vol. 28, no. 5, 1 March 2000 (2000-03-01), pages 1059-1066, XP002164138, ISSN: 0305-1048, DOI: 10.1093/NAR/28.5.1059

## Description

### FIELD OF THE INVENTION

The present invention is in the field of molecular biology, in particular in the field of enzymes and more particular in the field of polymerases. It is also in the field of nucleic acid sequencing.

### BACKGROUND

The invention relates to polymerase enzymes, in particular modified DNA polymerases which show improved incorporation of modified nucleotides compared to a control polymerase. Also included in the present invention are methods of using the modified polymerases for DNA sequencing, in particular next generation sequencing.

Three main super families of DNA polymerase exist, based upon their amino acid similarity to *E. coli* DNA polymerases I, II and III. They are called family A, B and C polymerases respectively. Whilst crystallographic analysis of Family A and B polymerases reveals a common structural core for the nucleotide binding site, sequence motifs that are well conserved within families are only weakly conserved between families, and there are significant differences in the way these polymerases discriminate between nucleotide analogues. Early experiments with DNA polymerases revealed difficulties incorporating modified nucleotides such as dideoxynucleotides (ddNTPs). There are, therefore, several examples in which DNA polymerases have been modified to increase the rates of incorporation of nucleotide analogues. The majority of these have focused on variants of Family A polymerases with the aim of increasing the incorporation of dideoxynucleotide chain terminators. For example, Tabor, S. and Richardson, C.C. ((1995) Proc. Natl. Acad. Sci (USA) 92:6339) describe the replacement of phenylalanine 667 with tyrosine in T. aquaticus DNA polymerase and the effects this has on discrimination of dideoxynucleotides by the DNA polymerase.

In order to increase the efficiency of incorporation of modified nucleotides, DNA polymerases have been utilised or engineered such that they lack 3 ' -5 ' exonuclease activity (designated exo-). The exo- variant of 9°N polymerase is described by Perler et al., 1998 US 5756334 and by Southworth et al., 1996 Proc. Natl Acad. Sci USA 93:5281.

Gardner A.F. and Jack W.E. (Determinants of nucleotide sugar recognition in an archaeon DNA polymerase Nucl. Acids Res. 27:2545, 1999) describe mutations in Vent DNA polymerase that enhance the incorporation of ribo-, 2' and 3'deoxyribo- and 2'-3'-dideoxy-ribonucleotides. The two individual mutations in Vent polymerase, Y412V and A488L, enhanced the relative activity of the enzyme with the nucleotide ATP. In addition, other substitutions at Y412 and A488 also increased ribonucleotide incorporation, though to a lesser degree. It was concluded that the bulk of the amino acid side chain at residue 412 acts as a "steric gate" to block access of the 2 '-hydroxyl of the ribonucleotide sugar to the binding site. However, the rate enhancement with cordycepin (3'deoxy adenosine triphosphate) was only 2- fold, suggesting that the Y412V polymerase variant was also sensitive to the loss of the 3 ' sugar hydroxyl. For residue A488, the change in activity is less easily rationalized. A488 is predicted to point away from the nucleotide binding site; here the enhancement in activity was explained through a change to the activation energy required for the enzymatic reaction. These mutations in Vent correspond to Y409 and A485 in 9°N polymerase.

The universality of the A488L mutation in conferring reduced discrimination against nucleotide analogs has been confirmed by homologous mutations in the following hyperthermophilic polymerases:
A486Y variant of Pfu DNA polymerase (Evans et al., 2000. Nucl. Acids. Res. 28:1059). A series of random mutations was introduced into the polymerase gene and variants were identified that had improved incorporation of ddNTPs. The A486Y mutation improved the ratio of ddNTP/dNTP in sequencing ladders by 150 -fold compared to wild type. However, mutation of Y410 to A or F produced a variant that resulted in an inferior sequencing ladder compared to the wild type enzyme. For further information, reference is made to International Publication No. WO 01/38546.

A485L variant of 9°N DNA polymerase (Gardner and Jack, 2002. Nucl. Acids Res. 30:605). This study demonstrated that the mutation of Alanine to Leucine at amino acid 485 enhanced the incorporation of nucleotide analogues that lack a 3' sugar hydroxyl moiety (acyNTPs and dideoxyNTPs).

A485T variant of Tsp JDF-3 DNA polymerase (Arezi et al., 2002. J. Mol. Biol. 322:719). In this paper, random mutations were introduced into the JDF-3 polymerase from which variants were identified that had enhanced incorporation of ddNTPs. Individually, two mutations, A485T and P410L, improved ddNTP uptake compared to the wild type enzyme. In combination, these mutations had an additive effect and improved ddNTP incorporation by 250 -fold. This paper demonstrates that the simultaneous mutation of two regions of a DNA polymerase can have additive effects on nucleotide analogue incorporation. In addition, this report demonstrates that P410, which lies adjacent to Y409 described above, also plays a role in the discrimination of nucleotide sugar analogues.

WO 01/23411 describes the use of the A488L variant of Vent in the incorporation of dideoxynucleotides and acyclonucleotides into DNA. The application also covers methods of sequencing that employ these nucleotide analogues and variants of 9°N DNA polymerase that are mutated at residue 485.

WO 2005/024010 A1 also relates to the modification of the motif A region and to the 9°N DNA polymerase. EP 1 664 287 B1 also relates to various altered family B type archeal polymerase enzymes which is capable of improved incorporation of nucleotides which have been modified at the 3' sugar hydroxyl such that the substituent is larger in size than the naturally occurring 3' hydroxyl group, compared to a control family B type archeal polymerase enzyme.

Yet, the modifications today still do not show sufficiently high incorporation rates of modified nucleotides (3'OH substituted analogs or having both substitutions on 3'-OH and carrying labels at the base). It would therefore be beneficial in order to improve sequencing performance to have enzymes that have such high incorporation rates of variety of modified nucleotides. One additional feature that is desirable is the tolerance for base modifications. For example, labels can be attached to the base or the 3'-OH via cleavable or non-cleavable linkers. In case of cleavable linkers attached to the base, there is usually a residual spacer arm left after the cleavage. This residual modification may interfere with incorporation of subsequent nucleotides by polymerase. Therefore, it is highly desirable to have polymerases for carrying out sequencing by synthesis process (SBS) that are tolerable of these scars.

### SUMMARY OF THE INVENTION

To improve the efficiency of certain DNA sequencing methods, the inventors have attempted to look for organisms other than 9°N. Also, to improve the efficiency of certain DNA sequencing methods, the inventors have analyzed whether such other DNA polymerases could be modified to produce improved rates of incorporation of such 3' substituted nucleotide analogues.

The disclosure relates to a polymerase enzyme according to SEQ ID NO. 1 or any polymerase that shares at least 70%, 80%, 90%, 95%, 98% amino acid sequence identity thereto, comprising a mutation selected from the group of: (i) at position 409 of SEQ ID NO. 1: serine (S) and/or (L409S), (ii) at position 410 of SEQ ID NO. 1: glycine (G) and/or (Y410G), (iii) at position 411 of SEQ ID NO. 1: serine (S) (P411S), wherein the enzyme has little or no 3'-5' exonuclease activity. Preferably, the enzyme is from *Pyrococcus furiosus.* In one embodiment polymerases also carry modifications/substitutions at position equivalent to 486 of SEQ ID NO. 1. Particularly preferred substitution is A->L. Substitutions at this position exhibit synergy with substitutions at positions 409/410/411.

The invention also relates to the use of a modified polymerase in DNA sequencing and a kit comprising such an enzyme.

Herein, "incorporation" means joining of the modified nucleotide to the free 3' hydroxyl group of a second nucleotide via formation of a phosphodiester linkage with the 5' phosphate group of the modified nucleotide. The second nucleotide to which the modified nucleotide is joined will typically occur at the 3' end of a polynucleotide chain.

Herein, "modified nucleotides" and "nucleotide analogues" when used in the context of this invention refer to nucleotides which have been modified at the 3' sugar hydroxyl such that the substituent is larger in size than the naturally occurring 3' hydroxyl group. In addition, these nucleotides may carry additional modifications, such as detectable labels attached to the base moiety. These terms may be used interchangeably.

Herein, the term "large 3' substituent(s)" refers to a substituent group at the 3' sugar hydroxyl which is larger in size than the naturally occurring 3' hydroxyl group.

Herein, "improved" incorporation is defined to include an increase in the efficiency and/or observed rate of incorporation of at least one modified nucleotide, compared to a control polymerase enzyme. However, the invention is not limited just to improvements in absolute rate of incorporation of the modified nucleotides. As shown below the polymerases also incorporate other modifications and so called dark nucleotides nucleotides (non-labeled, terminating or reversibly terminating), hence, "improved incorporation" is to be interpreted accordingly as also encompassing improvements in any of these other properties, with or without an increase in the rate of incorporation. For example, tolerance for modifications on the bases could be the result of the improved properties as could be ability to incorporate modified nucleotides at a range of concentrations and temperatures. The "improvement" need not be constant over all cycles. Herein, "improvement" may be the ability to incorporate the modified nucleotides at low temperatures and/or over a wider temperature range than the control enzyme. Herein, "improvement" may be the ability to incorporate the modified nucleotides when using a lower concentration of the modified nucleotides as substrate or lower concentration of polymerase. Preferably the altered polymerase should exhibit detectable incorporation of the modified nucleotide when working at a substrate concentration in the nanomolar range.

Herein, "altered polymerase enzyme" means that the polymerase has at least one amino acid change compared to the control polymerase enzyme. In general, this change will comprise the substitution of at least one amino acid for another. In certain instances, these changes will be conservative changes, to maintain the overall charge distribution of the protein. However, the invention is not limited to only conservative substitutions. Non-conservative substitutions are also envisaged in the present invention. Moreover, it is within the contemplation of the present invention that the modification in the polymerase sequence may be a deletion or addition of one or more amino acids from or to the protein, provided that the polymerase has improved activity with respect to the incorporation of nucleotides modified at the 3' sugar hydroxyl such that the substituent is larger in size than the naturally occurring 3' hydroxyl group as compared to a control polymerase enzyme, such as P. furiosus wildtype (SEQ ID NO. 1), however lacking the 3'-5' exonuclease activity.

The control polymerase may comprise any one of the listed substitution mutations functionally equivalent to the amino acid sequence of the given base polymerase (or an exo-variant thereof). Thus, the control polymerase may be a mutant version of the listed base polymerase having one of the stated mutations or combinations of mutations, and preferably having amino acid sequence identical to that of the base polymerase (or an exo- variant thereof) other than at the mutations recited above. Alternatively, the control polymerase may be a homologous mutant version of a polymerase other than the stated base polymerase, which includes a functionally equivalent or homologous mutation (or combination of mutations) to those recited in relation to the amino acid sequence of the base polymerase. By way of illustration, the control polymerase could be a mutant version of the Pfu polymerase having one of the mutations or combinations of mutations listed as optional or preferable above and below relative to the Pfu amino acid sequence, or it could be a mutant version of another polymerase. It would however not comprise the S-G-S mutation claimed herein.

The invention also encompasses enzymes claimed herein, wherein the amino acid sequence has been altered in non-conserved regions or positions. One skilled in the art will understand that many amino acid positions may be altered without changing the enzyme activity.

As used herein, the term, "nucleotide" comprises a purine or pyrimidine base linked glycosidically to a sugar (ribose or deoxyribose), and one or more phosphate groups attached to the 5' position of the sugar. "Nucleosides", as used herein, comprise a purine or pyrimidine base linked glycosidically to a sugar (ribose or deoxyribose), but lack a phosphate group at the 5' position of the sugar. With respect to the method claims described herein, it is generally understood that a nucleoside (lacking a 5' phosphate group) cannot be incorporated by a polymerase. Synthetic and naturally occurring nucleotides, prior to their modification at the 3' sugar hydroxyl, are included within the definition. Labeling of the bases can occur via naturally occurring groups (such as exocyclic amines for adenosine or guanosine) or via modifications, such as 5- and 7- deaza analogs. One preferred embodiment is attachment via 5- (pyrimidines) and 7- deaza (purines) propynyl group, more preferably propargylamine or propargylhydroxy group. Another preferred attachment is via hydroxymethyl groups as disclosed in US9322050.

Herein, and throughout the specification mutations within the amino acid sequence of a polymerase are written in the following form: (i) single letter amino acid as found in wild type polymerase, (ii) position of the change in the amino acid sequence of the polymerase and (iii) single letter amino acid as found in the altered polymerase. So, mutation of a Tyrosine residue in the wild type polymerase to a Valine residue in the altered polymerase at position 410 of the amino acid sequence would be written as Y410V. This is standard procedure in molecular biology.

### DETAILED DESCRIPTION OF THE INVENTION

The sheer increase in rates of incorporation of the modified analogues that have been achieved with polymerases of the invention is unexpected. The examples show that even existing polymerases with mutations do not exhibit these high incorporation rates.

The invention relates to a polymerase enzyme according to SEQ ID NO. 1 or any polymerase that shares at least 70%, 80%, 85%, 90%, 95% or, 98% amino acid sequence identity thereto, comprising a mutation selected from the group of: (i) at position 409 of SEQ ID NO. 1: serine (S) (L409S) and, (ii) at position 410 of SEQ ID NO. 1: glycine (G) (Y410G) and, (iii) at position 411 of SEQ ID NO. 1: serine (S) (P411S), wherein the enzyme has little or no 3'-5' exonuclease activity.

Preferably, the enzyme claimed shares 75%, 80%, 85%, 90%, 95%, 98%, 99%, 99,5% or 100% sequence identity with the enzyme according to SEQ ID NO. 1. These percentages do not include the additionally claimed mutations.

The invention also relates to a nucleic acid encoding an enzyme according to SEQ ID NO. 2, in particular with a DNA sequence according to SEQ ID NO. 3.

The altered polymerase will generally and preferably be an "isolated" or "purified" polypeptide. By "isolated polypeptide" a polypeptide that is essentially free from contaminating cellular components is meant, such as carbohydrates, lipids, nucleic acids or other proteinaceous impurities which may be associated with the polypeptide in nature. One may use a His-tag for purification, but other means may also be used. Preferably, at least the altered polymerase may be a "recombinant" polypeptide.

The altered polymerase according to the invention may be a family B type DNA polymerase, or a mutant or variant thereof. Family B DNA polymerases include numerous archaeal DNA polymerase, human DNA polymerase α and T4, RB69 and ϕ29 phage DNA polymerases. Family A polymerases include polymerases such as Taq, and T7 DNA polymerase. In one embodiment the polymerase is selected from any family B archaeal DNA polymerase, human DNA polymerase α or T4, RB69 and ϕ29 phage DNA polymerases.

Preferably, the polymerase is from an organism belonging to the family of Thermococcaceae, preferably from the genera of Pyrococcus. Such organisms include, Pyrococcus abyssi, Pyrococcus woesei, Pyrococcus yayanosii, Pyrococcus horikoshii, Pryococcus furiosus or, e.g. Pryococcus glycovorans. The most preferred is Pyrococcus furiosus.

Ideally, the polymerase comprises all of the following mutations, L408S, Y409G and P410S and optionally additionally, comprises one or more of the following additional mutations (numbering for 9°N) or equivalent mutations in other polymerase families: D141A, E143A, A485L. Mutations at 141/143 positions are known to eliminate most of the exonuclease proofreading ability. Mutations at position 485 (9°N) are known to enhance incorporation of non-native nucleotides (terminator mutations); see Gardner and Jack, 2002. Nucl. Acids Res. 30:605.

Preferably, the enzyme additionally comprises a mutation A486L in SEQ ID NO. 1.

Preferred is a polymerase, wherein the enzyme shares 95%, preferably even 98% sequence identity (not counting the mutations) with SEQ ID NO. 1 and additionally has the following set of mutations, (i) L409S, Y410G, P41 IS and (ii) A486L.

Preferred is a polymerase, wherein the enzyme shares 95%, preferably even 98% sequence identity with SEQ ID NO. 2 (JPol122).

Preferred is a polymerase, wherein the enzyme shares 95%, preferably even 98% sequence identity with SEQ ID NO. 2. In a very preferred embodiment the enzyme as an amino acid sequence exactly according to SEQ ID NO. 2.

Preferably, the modified polymerase comprises a mutation corresponding to A485L in 9°N polymerase. This mutation corresponds to A488L in Vent and A486L in Pfu. Several other groups have published on this mutation. A486Y variant of Pfu DNA polymerase (Evans et al., 2000. Nucl. Acids. Res. 28:1059). A series of random mutations was introduced into the polymerase gene and variants were identified that had improved incorporation of ddNTPs. The A486Y mutation improved the ratio of ddNTP/dNTP in sequencing ladders by 150-fold compared to wild type. However, mutation of Y410 to A or F produced a variant that resulted in an inferior sequencing ladder compared to the wild type enzyme; see also WO 01/38546. A485L variant of 9°N DNA polymerase (Gardner and Jack, 2002. Nucl. Acids Res. 30:605). This study demonstrated that the mutation of Alanine to Leucine at amino acid 485 enhanced the incorporation of nucleotide analogues that lack a 3' sugar hydroxyl moiety (acyNTPs and dideoxyNTPs). A485T variant of Tsp JDF-3 DNA polymerase (Arezi et al., 2002. J. Mol. Biol. 322:719). In this paper, random mutations were introduced into the JDF-3 polymerase from which variants were identified that had enhanced incorporation of ddNTPs. WO 01/23411 describes the use of the A488L variant of Vent in the incorporation of dideoxynucleotides and acyclonucleotides into DNA. The application also covers methods of sequencing that employ these nucleotide analogues and variants of 9°N DNA polymerase that are mutated at residue 485.

The invention relates to a polymerase with the mutations shown herein which exhibits an increased rate of incorporation of nucleotides which have been modified at the 3' sugar hydroxyl such that the substituent is larger in size than the naturally occurring 3' hydroxyl group and ddNTP, compared to the control polymerase being a normal unmodified enzyme.

Such nucleotides are disclosed in WO 2004/018497 A2. Here, a modified nucleotide molecule comprising a purine or pyrimidine base and a ribose or deoxyribose sugar moiety having a removable 3'-OH blocking group covalently attached thereto, such that the 3' carbon atom has attached a group of the structure: -O-Z is disclosed, wherein Z is any of -C(R')₂-N(R")₂'C(R')₂-N(H)R", and -C(R')₂-N₃, wherein each R" is or is part of a removable protecting group; each R' is independently a hydrogen atom, an alkyl, substituted alkyl, arylalkyl, alkenyl, alkynyl, aryl, heteroaryl, heterocyclic, acyl, cyano, alkoxy, aryloxy, heteroaryloxy or amido group, or a detectable label attached through a linking group; or (R')₂ represents an alkylidene group of formula =C(R"')₂ wherein each R'" may be the same or different and is selected from the group comprising hydrogen and halogen atoms and alkyl groups; and wherein said molecule may be reacted to yield an intermediate in which each R" is exchanged for H, which intermediate dissociates under aqueous conditions to afford a molecule with a free 3'OH.

The inventors have found that the claimed polymerase may be used in extension reactions and sequencing reactions very well when a novel nucleotide is used. Thus, the invention relates to a method of sequencing a nucleic acid wherein the claimed polymerase is used together with the following nucleotide.

In a preferred embodiment nucleotide has the following characteristics. The nucleotide comprises a nucleobase, a sugar, and at least one phosphate group at the 5' position, wherein said nucleobase comprising a detectable label attached via a cleavable oxymethylenedisulfide linker, said sugar comprising a 3'-O capped by a cleavable protecting group comprising methylenedisulfide.3' -O

Ideally, the nucleobase is a non-natural nucleobase and is selected from the group comprising 7-deaza guanine, 7-deaza adenine, 2-amino,7-deaza adenine, and 2-amino adenine.

Ideally, the cleavable protecting group is of the formula -CH₂-SS-R, wherein R is selected from the group comprising alkyl and substituted alkyl groups.

Preferably, the nucleotide has this structure:

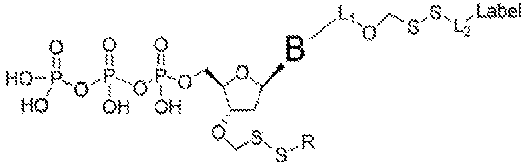

Here, B is a nucleobase, R is selected from the group comprising alkyl and substituted alkyl groups, and L1 and L2 are connecting groups. Preferably, L₁ and L₂ are independently selected from the group comprising -CO-, -CONH-, -NHCONH-, -0-, -S-, -ON, and -N=N-., alkyl, aryl, branched alkyl, branched aryl. Ideally L₁ and L₂ are the same.

The invention relates to a kit comprising a DNA polymerase as disclosed herein and claimed herein, and at least one nucleotide (e.g. a deoxynucleotide triphosphate) comprising a nucleobase, a sugar, and at least one phosphate group at the 5' position, wherein said sugar comprising a cleavable protecting group on the 3'-O, wherein said cleavable protecting group comprises methylenedisulfide, and wherein said nucleotide further comprises a detectable label attached via a cleavable oxymethylenedisulfide linker to the nucleobase of said nucleotide.

Claimed is also a reaction mixture comprising a nucleic acid template with a primer hybridized to said template, a DNA polymerase according to the invention and at least one nucleotide comprising a nucleobase, a sugar, and at least one phosphate group at the 5' position, wherein said sugar comprising a cleavable protecting group on the 3'-O, wherein said cleavable protecting group comprises methylenedisulfide, wherein said nucleotide further comprises a detectable label attached via a cleavable oxymethylenedisulfide linker to the nucleobase of said nucleotide.
Claimed is a method of performing a DNA synthesis reaction comprising the steps of a) providing a nucleic acid template with a primer hybridized to said template, the DNA polymerase according to the invention, at least one nucleotide comprising a nucleobase a sugar, and at least one phosphate group at the 5' position, wherein said sugar comprising a cleavable protecting group on the 3'-O, wherein said cleavable protecting group comprises methylenedisulfide, wherein said nucleotide further comprises a detectable label attached via a cleavable oxymethylenedisulfide linker to the nucleobase of said nucleotide, and b) subjecting said reaction mixture to conditions which enable a DNA polymerase catalyzed primer extension reaction.

The invention also relates to a method for analyzing a DNA sequence comprising the steps of a) providing a nucleic acid template with a primer hybridized to said template forming a primer/template hybridization complex, b) adding DNA polymerase according to the invention, and a first nucleotide comprising a nucleobase, a sugar, and at least one phosphate group at the 5' position, wherein said sugar comprising a cleavable protecting group on the 3'-O, wherein said cleavable protecting group comprises methylenedisulfide, wherein said nucleotide further comprises a first detectable label attached via a cleavable oxymethylenedisulfide linker to the nucleobase of said nucleotide, c) subjecting said reaction mixture to conditions which enable a DNA polymerase catalyzed primer extension reaction so as to create a modified primer/template hybridization complex, and d) detecting a said first detectable label of said nucleotide in said modified primer/template hybridization complex. The blocking group may be repeatedly removed and novel nucleotides added. These methods are known to the person skilled in the art. Here, differently labeled, 3'-O methylenedisulfide capped nucleotide compounds representing analogs of A, G, C and T or U are used in step b).

Ideally, step e) is performed by exposing said modified primer/template hybridization complex to a reducing agent. This can be TCEP.

In another embodiment the labelled nucleotide that is used is as follows.

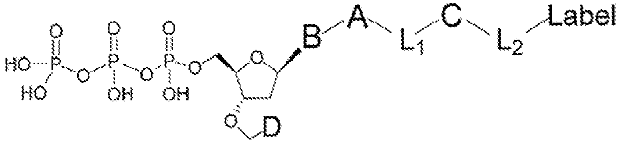

Here, D is selected from the group consisting of an azide, disulfide alkyl and disulfide substituted alkyl groups, B is a nucleobase, A is an attachment group, C is a cleavable site core, L₁ and L₂ are connecting groups, and Label is a label. Ideally, the nucleobase is selected from the group of 7-deaza guanine, 7-deaza adenine, 2-amino,7-deaza adenine, and 2-amino adenine.

L₁ is selected from the group consisting of -CONH(CH₂)ₓ- -CO-O(CH₂)ₓ- -CONH-(OCH₂CH₂0)ₓ-CO-O(CH₂CH₂0)ₓ- and -CO(CH₂)ₓ- wherein x is 0-10. L₂ can be,

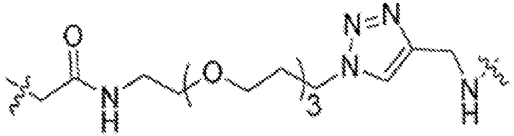

L₂ can be, -NH-, -(CH₂)_{X}-NH-, -C(Me)₂(CH₂)ₓNH-, -CH(Me)(CH₂)ₓNH-, -C(Me)₂(CH₂)ₓCO, -CH(Me)(CH₂)ₓCO-, -(CH₂)ₓOCONH(CH₂)_{y}O(CH₂)_{z}NH-, - (CH₂)ₓCONH(CH₂CH₂O)_{y}(CH₂)_{z}NH-, and -CONH(CH₂)ₓ-, -CO(CH₂)ₓ- wherein x, y, and z are each independently selected from is 0-10.

Preferably the labelled nucleotide has the following structure:

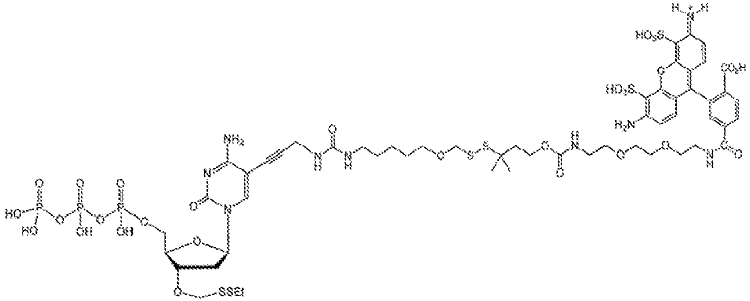

Preferably the labelled nucleotide has the following structure:

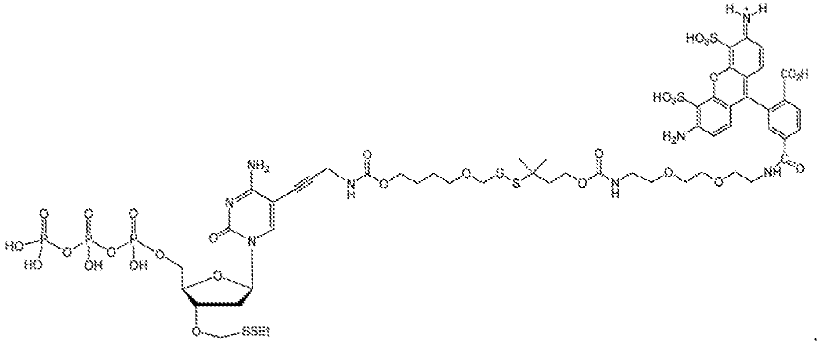

Preferably the labelled nucleotide has the following structure:

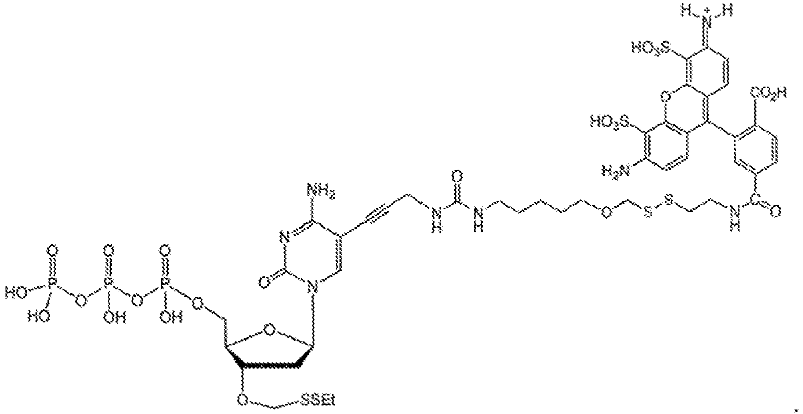

Preferably the labelled nucleotide has the following structure:

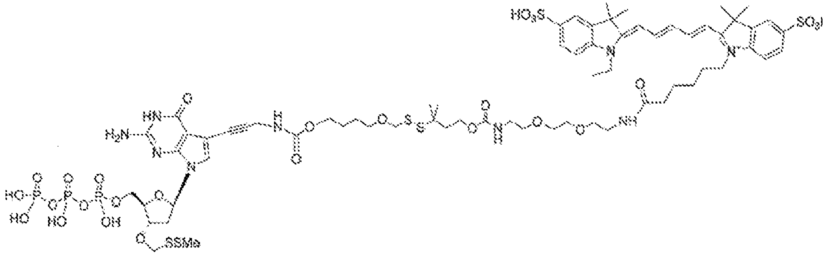

Preferably the labelled nucleotide has the following structure:

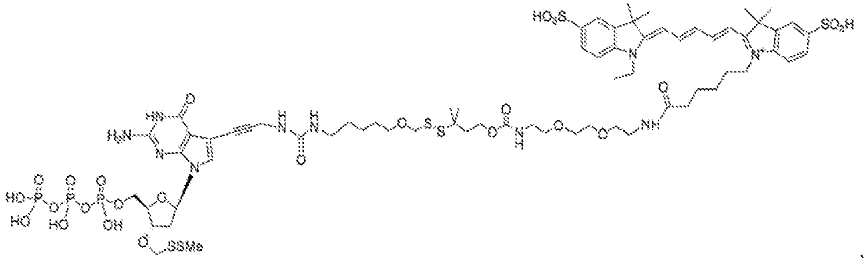

Preferably the labelled nucleotide has the following structure:

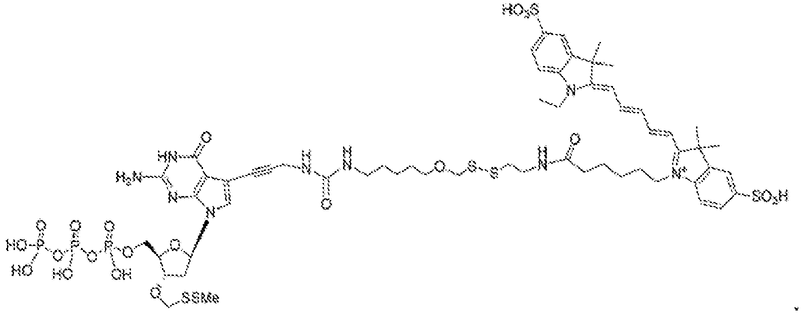

Preferably the labelled nucleotide has the following structure:

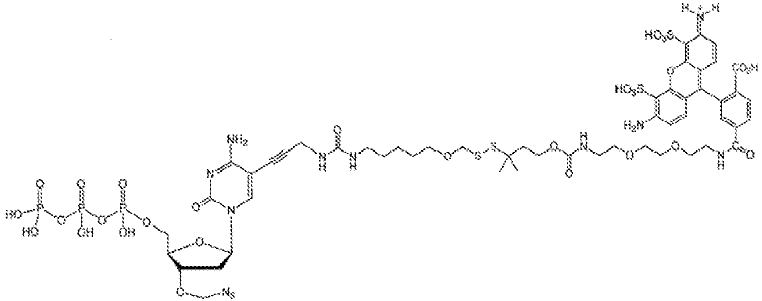

Preferably the labelled nucleotide has the following structure:

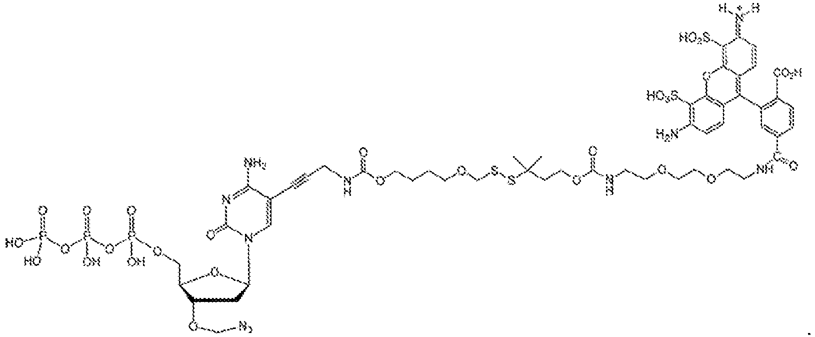

Preferably the labelled nucleotide has the following structure:

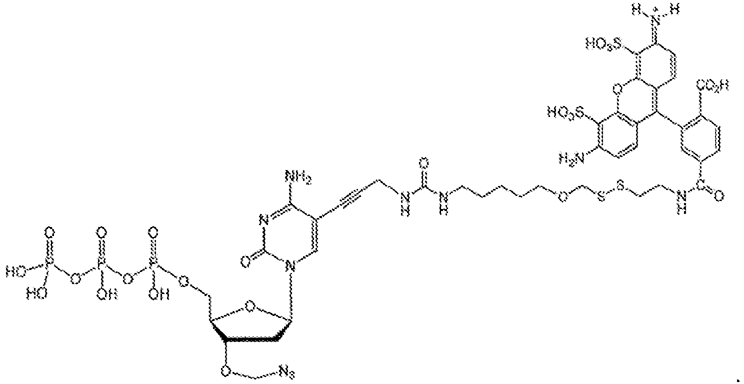

Preferably the labelled nucleotides have the following structures:

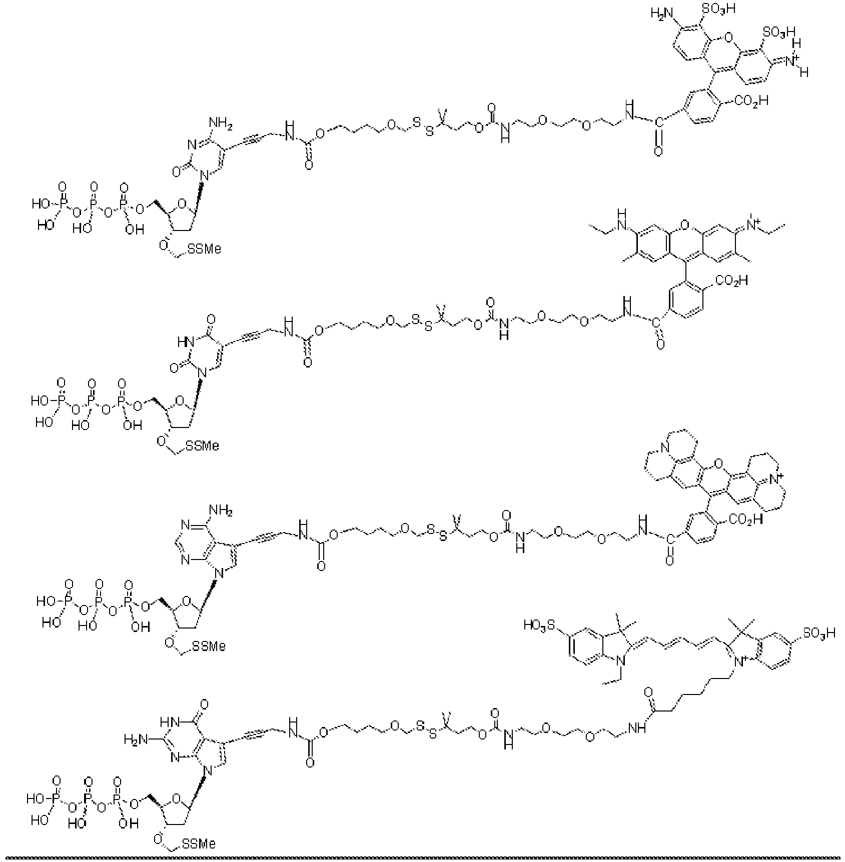

Preferably the non labelled nucleotides have the following structures:

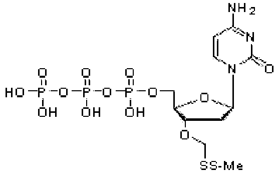

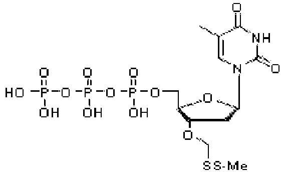

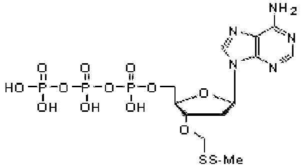

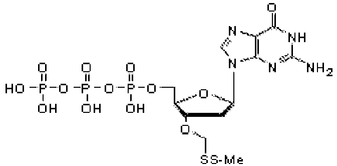

The invention also relates to a nucleic acid molecule encoding a polymerase according to the invention, as well as an expression vector comprising said nucleic acid molecule.

The invention also relates to a method for incorporating nucleotides which have been modified at the 3' sugar hydroxyl such that the substituent is larger in size than the naturally occurring 3' hydroxyl group into DNA comprising the following substances (i) a polymerase according to the invention, (ii) template DNA, (iii) one or more nucleotides, which have been modified at the 3' sugar hydroxyl such that the substituent is larger in size than the naturally occurring 3' hydroxyl group.

The invention also relates to a method for incorporating nucleotides which have been modified at the 3' sugar hydroxyl such that the substituent is larger in size than the naturally occurring 3' hydroxyl group into DNA comprising the following substances (i) a polymerase according to the invention, (ii) template DNA, (iii) one or more nucleotides, which have been modified at the 3' sugar hydroxyl such that the substituent is larger in size than the naturally occurring 3' hydroxyl group, wherein the blocking group comprises a disulfide preferably, methylenedisulfide.

The invention also relates to the use of a polymerase according to the invention in methods such as nucleic acid labeling, or sequencing. The polymerases of the present invention are useful in a variety of techniques requiring incorporation of a nucleotide into a polynucleotide, which include sequencing reactions, polynucleotide synthesis, nucleic acid amplification, nucleic acid hybridization assays, single nucleotide polymorphism studies, and other such techniques. All such uses and methods utilizing the modified polymerases of the invention are included within the scope of the present invention.

In sequencing the use of nucleotides bearing a 3' block allows successive nucleotides to be incorporated into a polynucleotide chain in a controlled manner. After each nucleotide addition the presence of the 3' block prevents incorporation of a further nucleotide into the chain. Once the nature of the incorporated nucleotide has been determined, the block may be removed, leaving a free 3' hydroxyl group for addition of the next nucleotide. Sequencing by synthesis of DNA ideally requires the controlled (i.e. one at a time) incorporation of the correct complementary nucleotide opposite the oligonucleotide being sequenced. This allows for accurate sequencing by adding nucleotides in multiple cycles as each nucleotide residue is sequenced one at a time, thus preventing an uncontrolled series of incorporations occurring. The incorporated nucleotide is read using an appropriate label attached thereto before removal of the label moiety and the subsequent next round of sequencing. In order to ensure only a single incorporation occurs, a structural modification ("blocking group") of the sequencing nucleotides is required to ensure a single nucleotide incorporation but which then prevents any further nucleotide incorporation into the polynucleotide chain. The blocking group must then be removable, under reaction conditions which do not interfere with the integrity of the DNA being sequenced. The sequencing cycle can then continue with the incorporation of the next blocked, labelled nucleotide. In order to be of practical use, the entire process should consist of high yielding, highly specific chemical and enzymatic steps to facilitate multiple cycles of sequencing. To be useful in DNA sequencing, a nucleotide, and more usually nucleotide triphosphates, generally require a 3 OH-blocking group so as to prevent the polymerase used to incorporate it into a polynucleotide chain from continuing to replicate once the base on the nucleotide is added. The DNA template for a sequencing reaction will typically comprise a double-stranded region having a free 3' hydroxyl group which serves as a primer or initiation point for the addition of further nucleotides in the sequencing reaction. The region of the DNA template to be sequenced will overhang this free 3' hydroxyl group on the complementary strand. The primer bearing the free 3' hydroxyl group may be added as a separate component (e.g. a short oligonucleotide) which hybridizes to a region of the template to be sequenced. Alternatively, the primer and the template strand to be sequenced may each form part of a partially self-complementary nucleic acid strand capable of forming an intramolecular duplex, such as for example a hairpin loop structure. Nucleotides are added successively to the free 3' hydroxyl group, resulting in synthesis of a polynucleotide chain in the 5' to 3' direction. After each nucleotide addition the nature of the base which has been added will be determined, thus providing sequence information for the DNA template.

Such DNA sequencing may be possible if the modified nucleotides can act as chain terminators. Once the modified nucleotide has been incorporated into the growing polynucleotide chain complementary to the region of the template being sequenced there is no free 3'-OH group available to direct further sequence extension and therefore the polymerase can not add further nucleotides. Once the nature of the base incorporated into the growing chain has been determined, the 3' block may be removed to allow addition of the next successive nucleotide. By ordering the products derived using these modified nucleotides it is possible to deduce the DNA sequence of the DNA template. Such reactions can be done in a single experiment if each of the modified nucleotides has attached a different label, known to correspond to the particular base, to facilitate discrimination between the bases added at each incorporation step. Alternatively, a separate reaction may be carried out containing each of the modified nucleotides separately.

In a preferred embodiment the modified nucleotides carry a label to facilitate their detection. Preferably this is a fluorescent label. Each nucleotide type may carry a different fluorescent label. However, the detectable label need not be a fluorescent label. Any label can be used which allows the detection of the incorporation of the nucleotide into the DNA sequence.

One method for detecting the fluorescently labelled nucleotides, suitable for use in the second and third aspects of the invention, comprises using laser light of a wavelength specific for the labelled nucleotides, or the use of other suitable sources of illumination.

In one embodiment the fluorescence from the label on the nucleotide may be detected by a CCD camera.

If the DNA templates are immobilised on a surface they may preferably be immobilised on a surface to form a high density array. Most preferably, and in accordance with the technology developed by the applicants for the present invention, the high density array comprises a single molecule array, wherein there is a single DNA molecule at each discrete site that is detectable on the array. Single-molecule arrays comprised of nucleic acid molecules that are individually resolvable by optical means and the use of such arrays in sequencing are described, for example, in WO 00/06770, the contents of which are incorporated herein by reference. Single molecule arrays comprised of individually resolvable nucleic acid molecules including a hairpin loop structure are described in WO 01/57248, the contents of which are also incorporated herein by reference. The polymerases of the invention are suitable for use in conjunction with single molecule arrays prepared according to the disclosures of WO 00/06770 of WO 01/57248. However, it is to be understood that the scope of the invention is not intended to be limited to the use of the polymerases in connection with single molecule arrays. Single molecule array-based sequencing methods may work by adding fluorescently labelled modified nucleotides and an altered polymerase to the single molecule array. Complementary nucleotides would base-pair to the first base of each nucleotide fragment and would be added to the primer in a reaction catalysed by the improved polymerase enzyme. Remaining free nucleotides would be removed. Then, laser light of a specific wavelength for each modified nucleotide would excite the appropriate label on the incorporated modified nucleotides, leading to the fluorescence of the label. This fluorescence could be detected by a suitable CCD camera that can scan the entire array to identify the incorporated modified nucleotides on each fragment. Thus millions of sites could potentially be detected in parallel. Fluorescence could then be removed. The identity of the incorporated modified nucleotide would reveal the identity of the base in the sample sequence to which it is paired. The cycle of incorporation, detection and identification would then be repeated approximately 25 times to determine the first 25 bases in each oligonucleotide fragment attached to the array, which is detectable. Thus, by simultaneously sequencing all molecules on the array, which are detectable, the first 25 bases for the hundreds of millions of oligonucleotide fragments attached in single copy to the array could be determined. Obviously the invention is not limited to sequencing 25 bases. Many more or less bases could be sequenced depending on the level of detail of sequence information required and the complexity of the array. Using a suitable bioinformatics program the generated sequences could be aligned and compared to specific reference sequences. This would allow determination of any number of known and unknown genetic variations such as single nucleotide polymorphisms (SNPs) for example. The utility of the altered polymerases of the invention is not limited to sequencing applications using single-molecule arrays. The polymerases may be used in conjunction with any type of array-based (and particularly any high density array-based) sequencing technology requiring the use of a polymerase to incorporate nucleotides into a polynucleotide chain, and in particular any array-based sequencing technology which relies on the incorporation of modified nucleotides having large 3' substituents (larger than natural hydroxyl group), such as 3' blocking groups. The polymerases of the invention may be used for nucleic acid sequencing on essentially any type of array formed by immobilisation of nucleic acid molecules on a solid support. In addition to single molecule arrays suitable arrays may include, for example, multi-polynucleotide or clustered arrays in which distinct regions on the array comprise multiple copies of one individual polynucleotide molecule or even multiple copies of a small-number of different polynucleotide molecules (e.g. multiple copies of two complementary nucleic acid strands). In particular, the polymerases of the invention may be utilised in the nucleic acid sequencing method described in WO 98/44152, the contents of which are incorporated herein by reference. This International application describes a method of parallel sequencing of multiple templates located at distinct locations on a solid support. The method relies on incorporation of labelled nucleotides into a polynucleotide chain. The polymerases of the invention may be used in the method described in International Application WO 00/18957, the contents of which are incorporated herein by reference. This application describes a method of solid-phase nucleic acid amplification and sequencing in which a large number of distinct nucleic acid molecules are arrayed and amplified simultaneously at high density via formation of nucleic acid colonies and the nucleic acid colonies are subsequently sequenced. The altered polymerases of the invention may be utilised in the sequencing step of this method. Multi-polynucleotide or clustered arrays of nucleic acid molecules may be produced using techniques generally known in the art. By way of example, WO 98/44151 and WO 00/18957 both describe methods of nucleic acid amplification which allow amplification products to be immobilised on a solid support in order to form arrays comprised of clusters or "colonies" of immobilised nucleic acid molecules. The contents of WO 98/44151 and WO 00/18957 relating to the preparation of clustered arrays and use of such arrays as templates for nucleic acid sequencing are incorporated herein by reference. The nucleic acid molecules present on the clustered arrays prepared according to these methods are suitable templates for sequencing using the polymerases of the invention. However, the invention is not intended to use of the polymerases in sequencing reactions carried out on clustered arrays prepared according to these specific methods. The polymerases of the invention may further be used in methods of fluorescent in situ sequencing, such as that described by Mitra et al. Analytical Biochemistry 320, 55-65, 2003.

Additionally, in another aspect, the invention provides a kit, comprising: (a) the polymerase according to the invention, and optionally, a plurality of different individual nucleotides of the invention and/or packaging materials therefor.

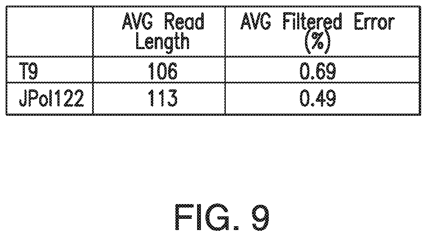

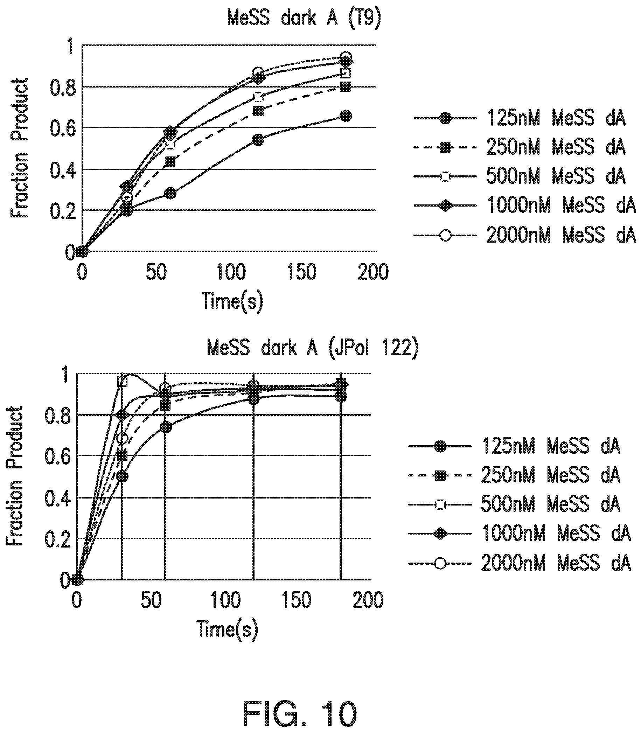

Several Experiments were carried out to show the increased rate of incorporation of nucleotides which have been modified compared to different wildtype polymerases and polymerases of the state of the art. Some of the results are shown in Figures 9 and 10. Further results with other wildtype polymerases and mutated polymerases from the state of the art also showed an increased rate of incorporation of nucleotides which have been modified as well as an enhanced specificity and sensitivity of the mutated polymerases according to the invention. The polymerases according to the invention show enhanced activity for incorporating bulky nucleotides also when compared to those disclosed in EP 1 664 287 B1.

### FIGURE CAPTIONS

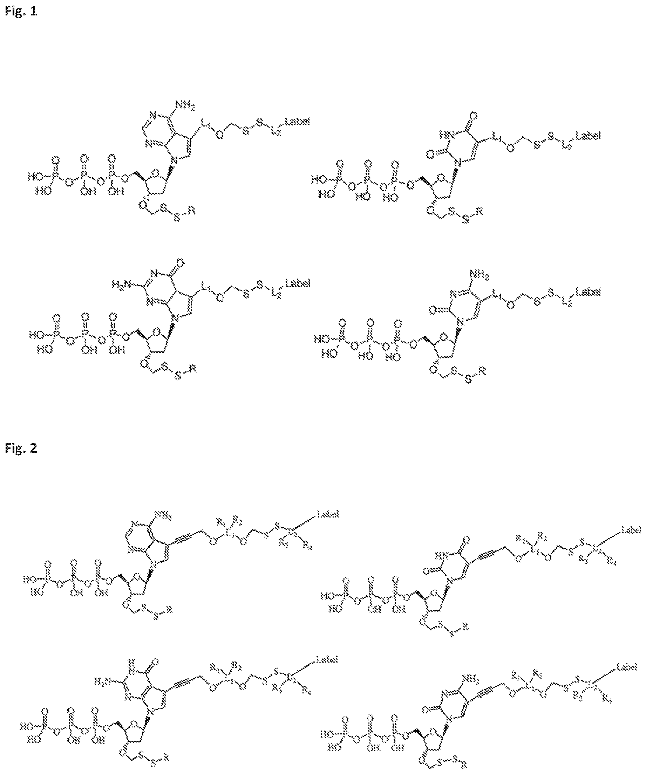

Fig. 1 shows labeled analogs of nucleotides with 3'-O methylenedisulfide-containing protecting group, where labels are attached to the nucleobase via cleavable oxymethylenedisulfide linker (-OCH₂-SS-). The analogs are (clockwise from the top left) for deoxyadenosine, thymidine or deoxyuridine, deoxycytidine and deoxyguanosine.
Fig. 2 shows an example of the labeled nucleotides where the spacer of the cleavable linker includes the propargyl ether linker. The analogs are (clockwise from the top left) for deoxyadenosine, thymidine or deoxyuridine, deoxycytidine and deoxyguanosine.
Fig. 3 shows a synthetic route of the labeled nucleotides specific for labeled dT intermediate.
Fig. 4 shows a cleavable linker synthesis starting from an 1,4-butanediol.

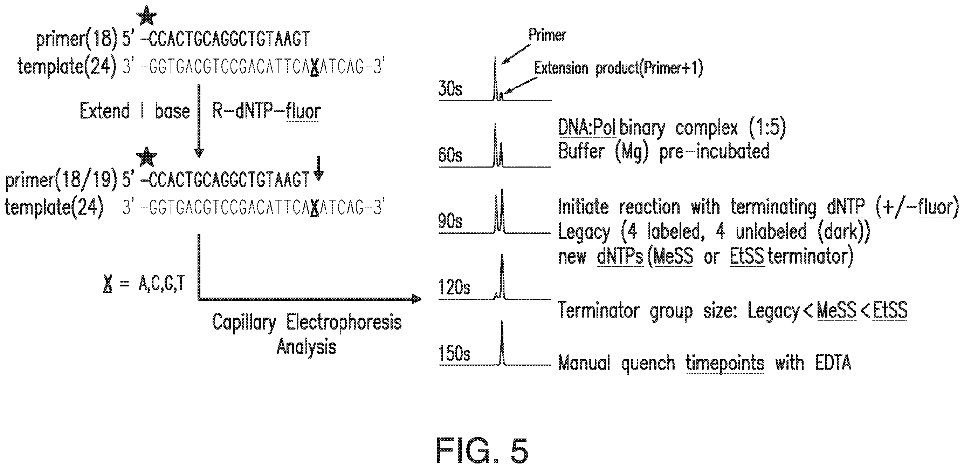

Fig. 5 shows the measurement of polymerase performance using extension in solution and capillary electrophoresis. The rate of single base terminating dNTP incorporation is measured. The extended fluorescent primer is detected by capillary electrophoresis (CE). The relative rate dNTP addition is determined by plots of fraction extended primer over time.

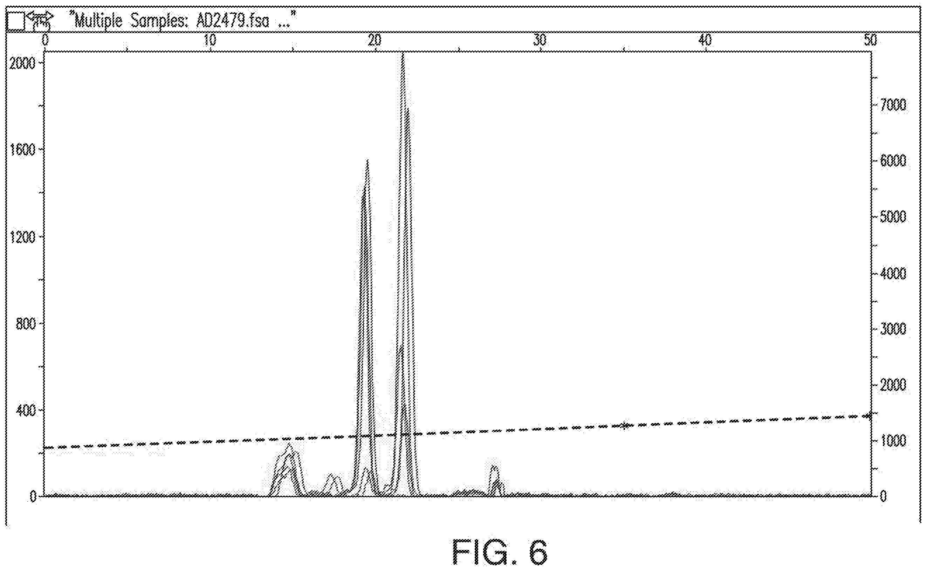

Fig. 6 shows kinetics of incorporation of nucleotide analogs (reversibly terminating dG) as measured by capillary electrophoresis assay. The methodology used here is solution based assay using synthetic DNA template and synthetic primer labeled with fluorophore at 5'end. The template is specific to the nucleotide interrogated. A mixture of pre-annealed primer/template, polymerase and nucleotide are incubated at temperature appropriate for the polymerase studied. After incubation an aliquot is loaded onto capillary electrophoresis system where size separation is performed using denaturing conditions and fluorescence detection. Peaks corresponding to non-extended primer, extended primer and residual nuclease activity (primer degradation) are observed in this trace indicating polymerase ability to incorporate nucleotide analog.

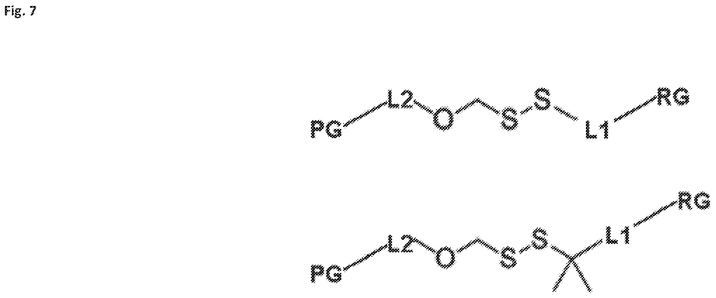

Fig. 7 shows generic universal building blocks structures comprising new cleavable linkers usable with the enzymes of the present invention. PG = Protective Group, LI, L2 - linkers (aliphatic, aromatic, mixed polarity straight chain or branched). RG = Reactive Group. In one embodiment of present invention such building blocks carry an Fmoc protective group on one end of the linker and reactive NHS carbonate or carbamate on the other end. This preferred combination is particularly useful in modified nucleotides synthesis comprising new cleavable linkers. A protective group should be removable under conditions compatible with nucleic acid/nucleotides chemistry and the reactive group should be selective. After reaction of the active NHS group on the linker with amine terminating nucleotide, an Fmoc group can be easily removed using base such as piperidine or ammonia, therefore exposing amine group at the terminal end of the linker for the attachment of cleavable marker. A library of compounds comprising variety of markers can be constructed this way very quickly.

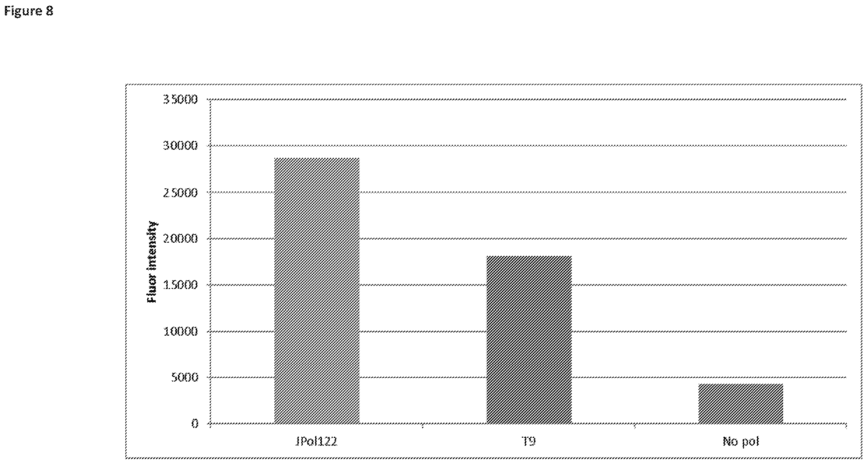

Fig. 8 shows incorporation of fluorescently labeled, reversibly terminating nucleotide Alexa488-dC- 3'-O-CH₂SSCH₃ as measured by fluorescence plate based assay for JPol122 and T9. Duplex DNA was immobilized on the plate, a solution of polymerase and nucleotide was added and after incubation plate was washed and read with fluorescence plate reader (exc. 490 nm/em. 520 nm).
Figure 9 shows performance of Jpol 122 (SEQ ID NO. 2) as measured by sequencing KPIs and compared to legacy (T9). The data shows significant improvement in average reads length and error rate for JPol122 (SEQ ID NO. 2).
Fig. 10 shows kinetics of incorporation of 3'-O-CH2-SS-CH3 dA nucleotide at various concentrations by polymerase T9 and JPol122 (SEQ ID NO. 2). The data shows much faster incorporation of this reversibly terminating nucleotide by JPol122 (SEQ ID NO. 2).

### EXAMPLES

### Enzyme Sequences

| | |
|---|---|
| **SEQ ID NO. 1** | |
| Wild Type LOCUS WP_ 011011325 775 aa linear BCT 24-MAY-2013 DEFINITION DNA polymerase [Pyrococcus furiosus]. ACCESSION WP_ 011011325 VERSION WP_011011325.1 | |
| **SEQ ID NO. 2** | |
| >JPol122_ | |
| P.furiosous (amino acid sequence) | |
| **SEQ ID NO. 3** | |
| >JPol122_ | |
| P.furiosous (DNA sequence) | |
| | |
| | |

### EXAMPLE 1

### Synthesis of 3'-O-(methylthiomethyl)-5'-O-(tert-butyldimethylsilyl)-2'-deoxythymidine (2)

5'-0-(tert-butyldimethylsilyl)-2'-deoxythymidine (1) (2.0g, 5.6 mmol) was dissolved in a mixture consisting of DMSO (10.5 mL), acetic acid (4.8 mL), and acetic anhydride (15.4 mL) in a 250 mL round bottom flask, and stirred for 48 hours at room temperature. The mixture was then quenched by adding saturated K₂CO₃ solution until evolution of gaseous CO₂ was stopped. The mixture was then extracted with EtOAc (3X100 mL) using a separating funnel. The combined organic extract was then washed with a saturated solution of NaHC0₃ (2X150 mL) in a partitioning funnel, and the organic layer was dried over Na₂S0₄. The organic part was concentrated by rotary evaporation. The reaction mixture was finally purified by silica gel column chromatography.

### EXAMPLE 2

### Synthesis of 3'-O-(ethyldithiomethyl)-2'-deoxythymidine (4)

Compound 2 (1.75g, 4.08 mmol), dried overnight under high vacuum, dissolved in 20 mL dry CH₂Cl₂ was added with EtsN (0.54 mL, 3.87 mmol) and 5.0g molecular sieve-3A, and stirred for 30 min under Ar atmosphere. The reaction flask was then placed on an ice-bath to bring the temperature to sub-zero, and slowly added with 1.8 eq 1M SO₂Cl₂ in CH₂Cl₂ (1.8 mL) and stirred at the same temperature for 1.0 hour. Then the ice-bath was removed to bring the flask to room temperature, and added with a solution of potassium thiotosylate (1.5 g) in 4 mL dry DMF and stirred for 0.5 hour at room temperature.

Then 2 eq EtSH (0.6 mL) was added and stirred additional 40 min. The mixture was then diluted with 50 mL CH₂Cl₂ and filtered through celite-S in a funnel. The sample was washed with adequate amount of CH₂Cl₂ to make sure that the product was filtered out. The CH₂Cl₂ extract was then concentrated and purified by chromatography on a silica gel column (Hex:EtOAC/1:1 to 1:3, Rf =0.3 in Hex:EtOAc/1:1). The resulting crude product was then treated with 2.2g of NH₄F in 20 mL MeOH. After 36 hours, the reaction was quenched with 20 mL saturated NaHC0₃ and extracted with CH₂Cl₂ by partitioning. The CH₂Cl₂ part was dried over Na₂SO₄ and purified by chromatography (Hex:EtOAc/1:1 to 1:2).

### EXAMPLE 3

### Synthesis of the triphosphate of 3'-O-(ethyldithiomethyl)-2'-deoxythymidine (5)

In a 25 mL flask, compound 4 (0.268g, 0.769 mmol) was added with proton sponge (210 mg), equipped with rubber septum. The sample was dried under high vacuum for overnight. The material was then dissolved in 2.6 mL (MeO)₃PO under argon atmosphere. The flask, equipped with Ar-gas supply, was then placed on an ice-bath, stirred to bring the temperature to sub-zero. Then 1.5 equivalents of POCI₃ was added at once by a syringe and stirred at the same temperature for 2 hours under Argon atmosphere. Then the ice-bath was removed and a mixture consisting of tributylammonium-pyrophosphate (1.6g) and Bu₃N (1.45 mL) in dry DMF (6 mL) was prepared. The entire mixture was added at once and stirred for 10 min. The reaction mixture was then diluted with TEAB buffer (30 mL, 100 mM) and stirred for additional 3 hours at room temperature. The crude product was concentrated by rotary evaporation, and purified by CI 8 Prep HPLC (method: 0 to 5min 100%A followed by gradient up to 50%B over 72min, A = 50 mM TEAB and B = acetonitrile). After freeze drying of the target fractions, the semi-pure product was further purified by ion exchange HPLC using PL-SAX Prep column (Method: 0 to 5min 100%A, then gradient up to 70%B over 70min, where A = 15% acetonitrile in water, B = 0.85M TEAB buffer in 15% acetonitrile). Final purification was carried out by CI8 Prep HPLC as described above resulting in ∼ 25% yield of compound 5.

### EXAMPLE 4

### Synthesis of N⁴-Benzoyl-5'-O-(tert-butyldimethylsilyl)-3'-O-(methylthiomethyl)-2' deoxycytidine (7)

N⁴-benzoyl-5'-0-(*tert*-butyldimethylsilyl)-2'-deoxycytidine (6) (50 g, 112.2 mmol) was dissolved in DMSO (210mL) in a 2L round bottom flask. It was added sequentially with acetic acid (210 mL) and acetic anhydride (96 mL), and stirred for 48 h at room temperature. During this period of time, a complete conversion to product was observed by TLC (Rf = 0.6, EtOAc:hex/10:1 for the product).
The mixture was separated into two equal fractions, and each was transferred to a 2000mL beaker and neutralized by slowly adding saturated K₂CO₃ solution until CO₂ gas evolution was stopped (pH 8). The mixture was then extracted with EtOAc in a separating funnel. The organic part was then washed with saturated solution of NaHC0₃ (2X1L) followed by with distilled water (2X1L), then the organic part was dried over Na₂SO₄.
The organic part was then concentrated by rotary evaporation. The product was then purified by silica gel flash-column chromatography using puriflash column (Hex:EtOAc/1:4 to 1:9, 3 column runs, on 15um, HC 300g puriflash column) to obtain N⁴-benzoyl-5'-0-(tertbutyldimethylsilyl)-3'-O-(methylthiomethyl)-2'-deoxycytidine (7) as grey powder in 60% yield.

### EXAMPLE 5

### N⁴-Benzoyl-3'-0-(ethyldithiomethyl)-5'-0-(tert-butyldimethylsilyl)-2'-deoxycytidine (8)

N⁴-Benzoyl-5'-0-(tert-butyldimethylsilyl)-3'-O-(methylthiomethyl)-2'-deoxycytidine (7) (2.526g, 5.0 mmol) dissolved in dry CH₂Cl₂ (35 mL) was added with molecular sieve-3A (10g). The mixture was stirred for 30 minutes. It was then added with Et3N (5.5 mmol), and stirred for 20 minutes on an ice-salt-water bath. It was then added slowly with 1M SO₂CI₂ in CH₂Cl₂ (7.5 mL, 7.5 mmol) using a syringe and stirred at the same temperature for 2 hours under N2-atmosphere. Then benzenethiosulfonic acid sodium salt (1.6g, 8.0 mmol) in 8 mL dry DMF was added and stirred for 30 minutes at room temperature. Finally, EtSH was added (0.74 mL) and stirred additional 50 minutes at room temperature. The reaction mixture was filtered through celite-S, and washed the product out with CH₂CI₂. After concentrating the resulting CH₂Cl₂ part, it was purified by flash chromatography using a silica gel column (1:1 to 3:7/Hex:EtOAc) to obtain compound 8 in 54.4% yield.

### EXAMPLE 6

### N⁴-Benzoyl-3'-O-(ethyldithiomethyl)-2'-deoxycytidine (9)

N⁴-Benzoyl-3'-O-(ethyldithiomethyl)-5'-0-(tert-butyldimethylsilyl)-2'-deoxycytidine (8, 1.50g, 2.72 mmol) was dissolved in 50 mL THF. Then 1M TBAF in THF (3.3 mL) was added at ice-cold temperature under nitrogen atmosphere. The mixture was stirred for 1 hour at room temperature. Then the reaction was quenched by adding 1 mL MeOH, and solvent was removed after 10 minutes by rotary evaporation. The product was purified by silica gel flash chromatography using gradient 1:1 to 1:9/Hex:EtOAc to result in compound 9. Finally, the synthesis of compound 10 was achieved from compound 9 following the standard synthetic protocol described in the synthesis of compound 5.

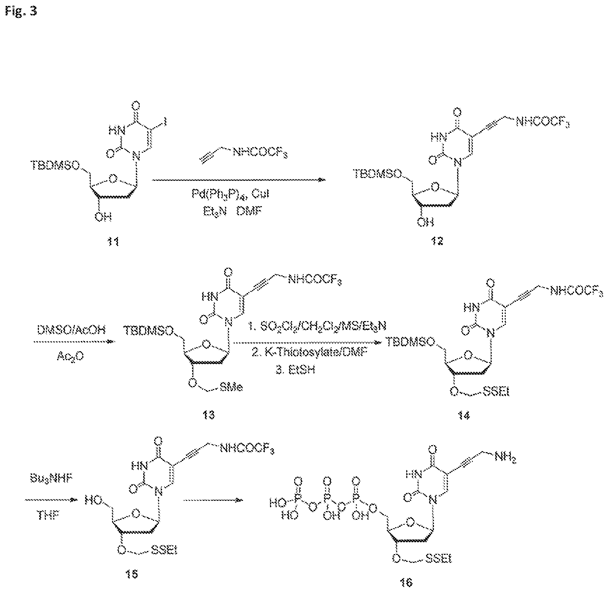

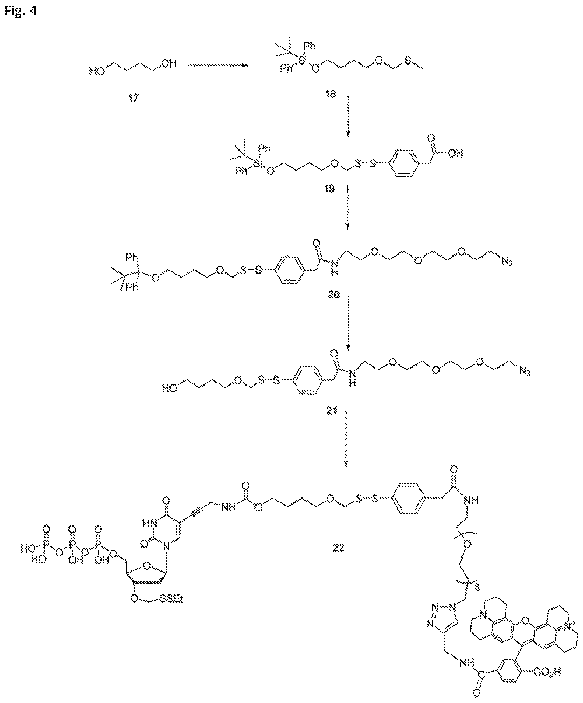

The synthesis of the labeled nucleotides can be achieved following the synthetic routes shown in Figure 3 and Figure 4. Figure 3 is specific for the synthesis of labeled dT intermediate, and other analogs could be synthesized similarly.

## Claims

1. Polymerase enzyme according to SEQ ID NO. 1 or any polymerase that shares at least 70%, 80%, 90%, 95% or, 98% amino acid sequence identity thereto, comprising the following mutation(s):
i. at position 409 of SEQ ID NO. 1: serine (S) (L409S) and/or
ii. at position 410 of SEQ ID NO. 1: glycine (G) (Y 410G) and/or
iii. at position 411 of SEQ ID NO. 1: serine (S) (P411S),
wherein the enzyme has little or no 3'-5' exonuclease activity, wherein the polymerase comprises all of the following mutations, L409S, Y410G and P411S and additionally, comprises one or more of the following additional mutations D141A, E143A, A486L.

2. The polymerase of claim 1, wherein the polymerase is from an organism belonging to the family of Thermococcaceae, preferably from the genera of Pyrococcus.

3. The polymerase according to claims 1 to 2, wherein the polymerase additionally comprises a mutation A486L.

4. The polymerase according to any of the preceding claims, wherein the enzyme shares 95%, preferably even 98% sequence identity with SEQ ID NO. 1 and additionally has the following set of mutations, (i) L409S, Y 410G, P411S and (ii) A486L.

5. The polymerase according to any of the preceding claims, wherein the enzyme has an amino acid sequence according to SEQ ID NO. 2.

6. The polymerase according to any preceding claim which exhibits an increased rate of incorporation of nucleotides which have been modified at the 3' sugar hydroxyl such that the substituent is larger in size than the naturally occurring 3' hydroxyl group, compared to the control polymerase.

7. A nucleic acid molecule encoding a polymerase according to claims 1 to 6, with a sequence according to SEQ ID NO. 3.

8. An expression vector comprising the nucleic acid molecule of claim 7.

9. A method for incorporating nucleotides which have been modified at the 3' sugar hydroxyl such that the substituent is larger in size than the naturally occurring 3' hydroxyl group into DNA comprising the following substances (i) a polymerase according to any one of claims 1 to 8, (ii) template DNA, (iii) one or more nucleotides, which have been modified at the 3' sugar hydroxyl such that the substituent is larger in size than the naturally occurring 3' hydroxyl group.

10. Use of a polymerase according to any of the claims 1 to 6 for DNA sequencing, DNA labeling, primer extension, amplification or the like.

11. Kit comprising a polymerase according to any of the claims 1 to 6.

## Patentansprüche

1. Polymeraseenzym gemäß SEQ ID NO. 1 oder eine beliebige Polymerase, die mindestens 70%, 80%, 90%, 95% oder 98% Aminosäuresequenzidentität dazu aufweist, umfassend die folgende(n) Mutation(en):
i. an Position 409 von SEQ ID NO. 1: Serin (S) (L409S) und/oder
ii. an Position 410 von SEQ ID NO. 1: Glycin (G) (Y410G) und/oder
iii. an Position 411 von SEQ ID NO. 1: Serin (S) (P411 S),
wobei das Enzym eine geringe oder keine 3'-5'- Exonukleaseaktivität aufweist, wobei die Polymerase alle folgenden Mutationen umfasst, L409S, Y41OG und P411S und zusätzlich eine oder mehrere der folgenden zusätzlichen Mutationen D141A, E143A, A486L umfasst.

2. Polymerase nach Anspruch 1, wobei die Polymerase aus einem Organismus der Familie der Thermococcaceae, vorzugsweise aus der Gattung von Pyrococcus, stammt.

3. Polymerase nach Anspruch 1 oder 2, wobei die Polymerase zusätzlich eine Mutation A486L umfasst.

4. Polymerase nach einem der vorhergehenden Ansprüche, wobei das Enzym 95%, vorzugsweise sogar 98% Sequenzidentität mit SEQ ID NO. 1 teilt und zusätzlich den folgenden Satz von Mutationen aufweist, (i) L409S, Y410G, P411S und (ii) A486L.

5. Polymerase nach einem der vorhergehenden Ansprüche, wobei das Enzym eine Aminosäuresequenz gemäß SEQ ID NO. 2 aufweist.

6. Polymerase nach einem der vorhergehenden Ansprüche, die eine erhöhte Inkorporationsrate von Nukleotiden zeigt, die an der 3'-Zuckerhydroxylgruppe so modifiziert wurden, dass der Substituent, im Vergleich zur Kontroll-Polymerase, größer als die natürlich vorkommende 3'-Hydroxylgruppe ist.

7. Nukleinsäuremolekül kodierend eine Polymerase gemäß den Ansprüchen 1 bis 6, mit einer Sequenz gemäß SEQ ID NO. 3.

8. Expressionsvektor umfassend das Nukleinsäuremolekül nach Anspruch 7.

9. Verfahren zum Einbau von Nukleotiden, die an der 3'-Zuckerhydroxylgruppe derart modifiziert wurden, dass der Substituent größer als die natürlich vorkommende 3'-Hydroxylgruppe ist, in DNA, umfassend die folgenden Substanzen (i) eine Polymerase nach einem der Ansprüche 1 bis 8, (ii) Matrizen-DNA, (iii) ein oder mehrere Nukleotide, die an der 3'-Zuckerhydroxylgruppe so modifiziert wurden, dass der Substituent größer als die natürlich vorkommende 3'-Hydroxylgruppe ist.

10. Verwendung einer Polymerase nach einem der Ansprüche 1 bis 6 zur DNA-Sequenzierung, DNA-Markierung, Primer-Verlängerung, Amplifikation oder dergleichen.

11. Kit enthaltend eine Polymerase nach einem der Ansprüche 1 bis 6.

## Revendications

1. Enzyme polymérase selon SEQ ID NO. 1 ou toute polymérase qui partage au moins 70%, 80%, 90%, 95% ou 98% d'identité de séquence d'acides aminés avec celle-ci, comprenant la ou les mutations suivantes:
i. à la position 409 de SEQ ID NO. 1: sérine (S) (L409S) et / ou
ii. à la position 410 de SEQ ID NO. 1: glycine (G) (Y 410G) et / ou
iii. à la position 411 de SEQ ID NO. 1: sérine (S) (P411S),
dans laquelle l'enzyme a peu ou pas d'activité d'exonucléase 3'-5',
dans laquelle la polymérase comprend toutes les mutations suivantes, L409S, Y 41 OG et P411S et comprend en outre une ou plusieurs des mutations supplémentaires suivantes D141A, E143A, A486L.

2. La polymérase selon la revendication 1, dans laquelle la polymérase provient d'un organisme appartenant à la famille des Thermococcaceae, de préférence du genre Pyrococcus.

3. La polymérase selon les revendications 1 à 2, dans laquelle la polymérase comprend en outre une mutation A486L.

4. La polymérase selon l'une quelconque des revendications précédentes, dans laquelle l'enzyme a une identité de séquence à 95%, de préférence même 98% avec SEQ ID NO. 1 et possède en outre l'ensemble suivant de mutations, (i) L409S, Y 410G, P411S et (ii) A486L.

5. La polymérase selon l'une quelconque des revendications précédentes, dans laquelle l'enzyme a une séquence d'acides aminés selon SEQ ID NO. 2.

6. La polymérase selon l'une quelconque des revendications précédentes, qui présente une grade accrue d'incorporation de nucléotides qui ont été modifiés au niveau du sucre hydroxyle 3 'de telle sorte que le substituant a une taille plus grande que le groupe hydroxyle 3' naturel, par rapport à la polymérase de contrôle.

7. Molécule d'acide nucléique codant pour une polymérase selon les revendications 1 à 6, avec une séquence selon SEQ ID NO. 3.

8. Vecteur d'expression comprenant la molécule d'acide nucléique selon la revendication 7.

9. Procédé pour incorporer des nucléotides qui ont été modifiés au niveau du sucre hydroxyle 3' de telle sorte que le substituant a une taille plus grande que le groupe hydroxyle 3' naturel dans l'ADN comprenant les substances suivantes (i) une polymérase selon l'une quelconque des revendications 1 à 8, (ii) ADN matrice, (iii) un ou plusieurs nucléotides, qui ont été modifiés au niveau du sucre hydroxyle 3 'de telle sorte que le substituant a une taille plus grande que le groupe hydroxyle 3' naturel.

10. Utilisation d'une polymérase selon l'une quelconque des revendications 1 à 6 pour le séquençage d'ADN, le marquage d'ADN, l'extension d'amorce, l'amplification ou analogue.

11. Kit comprenant une polymérase selon l'une quelconque des revendications 1 à 6.


### SEQUENCE LISTING

<110> QIAGEN Waltham Inc.
<120> POLYMERASE ENZYME FROM PYROCOCCUS FURIOSUS
<130> 37578.0052P1
<150> 62/458,397
   <151> 2017-02-13
<150> EP17160396
   <151> 2017-03-10
<160> 3
<170> PatentIn version 3.5
<210> 1
   <211> 775
   <212> PRT
   <213> Pyrococcus furiosus
<400> 1
   Met Ile Leu Asp Val Asp Tyr Ile Thr Glu Glu Gly Lys Pro Val Ile
   1               5                   10                  15
   Arg Leu Phe Lys Lys Glu Asn Gly Lys Phe Lys Ile Glu His Asp Arg
               20                  25                  30
   Thr Phe Arg Pro Tyr Ile Tyr Ala Leu Leu Arg Asp Asp Ser Lys Ile
           35                  40                  45
   Glu Glu Val Lys Lys Ile Thr Gly Glu Arg His Gly Lys Ile Val Arg
       50                  55                  60
   Ile Val Asp Val Glu Lys Val Glu Lys Lys Phe Leu Gly Lys Pro Ile
   65                  70                  75                  80
   Thr Val Trp Lys Leu Tyr Leu Glu His Pro Gln Asp Val Pro Thr Ile
                   85                  90                  95
   Arg Glu Lys Val Arg Glu His Pro Ala Val Val Asp Ile Phe Glu Tyr
               100                 105                 110
   Asp Ile Pro Phe Ala Lys Arg Tyr Leu Ile Asp Lys Gly Leu Ile Pro
           115                 120                 125
   Met Glu Gly Glu Glu Glu Leu Lys Ile Leu Ala Phe Asp Ile Glu Thr
       130                 135                 140
   Leu Tyr His Glu Gly Glu Glu Phe Gly Lys Gly Pro Ile Ile Met Ile
   145                 150                 155                 160
   Ser Tyr Ala Asp Glu Asn Glu Ala Lys Val Ile Thr Trp Lys Asn Ile
   165 170 175
   Asp Leu Pro Tyr Val Glu Val Val Ser Ser Glu Arg Glu Met Ile Lys
   180 185 190
   Arg Phe Leu Arg Ile Ile Arg Glu Lys Asp Pro Asp Ile Ile Val Thr
   195 200 205
   Tyr Asn Gly Asp Ser Phe Asp Phe Pro Tyr Leu Ala Lys Arg Ala Glu
   210 215 220
   Lys Leu Gly Ile Lys Leu Thr Ile Gly Arg Asp Gly Ser Glu Pro Lys
   225 230 235 240
   Met Gln Arg Ile Gly Asp Met Thr Ala Val Glu Val Lys Gly Arg Ile
   245 250 255
   His Phe Asp Leu Tyr His Val Ile Thr Arg Thr Ile Asn Leu Pro Thr
   260 265 270
   Tyr Thr Leu Glu Ala Val Tyr Glu Ala Ile Phe Gly Lys Pro Lys Glu
   275 280 285
   Lys Val Tyr Ala Asp Glu Ile Ala Lys Ala Trp Glu Ser Gly Glu Asn
   290 295 300
   Leu Glu Arg Val Ala Lys Tyr Ser Met Glu Asp Ala Lys Ala Thr Tyr
   305 310 315 320
   Glu Leu Gly Lys Glu Phe Leu Pro Met Glu Ile Gln Leu Ser Arg Leu
   325 330 335
   Val Gly Gln Pro Leu Trp Asp Val Ser Arg Ser Ser Thr Gly Asn Leu
   340 345 350
   Val Glu Trp Phe Leu Leu Arg Lys Ala Tyr Glu Arg Asn Glu Val Ala
   355 360 365
   Pro Asn Lys Pro Ser Glu Glu Glu Tyr Gln Arg Arg Leu Arg Glu Ser
   370 375 380
   Tyr Thr Gly Gly Phe Val Lys Glu Pro Glu Lys Gly Leu Trp Glu Asn
   385 390 395 400
   Ile Val Tyr Leu Asp Phe Arg Ala Leu Tyr Pro Ser Ile Ile Ile Thr
                   405                 410                 415
   His Asn Val Ser Pro Asp Thr Leu Asn Leu Glu Gly Cys Lys Asn Tyr
               420                 425                 430
   Asp Ile Ala Pro Gln Val Gly His Lys Phe Cys Lys Asp Ile Pro Gly
           435                 440                 445
   Phe Ile Pro Ser Leu Leu Gly His Leu Leu Glu Glu Arg Gln Lys Ile
       450                 455                 460
   Lys Thr Lys Met Lys Glu Thr Gln Asp Pro Ile Glu Lys Ile Leu Leu
   465                 470                 475                 480
   Asp Tyr Arg Gln Lys Ala Ile Lys Leu Leu Ala Asn Ser Phe Tyr Gly
                   485                 490                 495
   Tyr Tyr Gly Tyr Ala Lys Ala Arg Trp Tyr Cys Lys Glu Cys Ala Glu
               500                 505                 510
   Ser Val Thr Ala Trp Gly Arg Lys Tyr Ile Glu Leu Val Trp Lys Glu
           515                 520                 525
   Leu Glu Glu Lys Phe Gly Phe Lys Val Leu Tyr Ile Asp Thr Asp Gly
       530                 535                 540
   Leu Tyr Ala Thr Ile Pro Gly Gly Glu Ser Glu Glu Ile Lys Lys Lys
   545                 550                 555                 560
   Ala Leu Glu Phe Val Lys Tyr Ile Asn Ser Lys Leu Pro Gly Leu Leu
                   565                 570                 575
   Glu Leu Glu Tyr Glu Gly Phe Tyr Lys Arg Gly Phe Phe Val Thr Lys
               580                 585                 590
   Lys Arg Tyr Ala Val Ile Asp Glu Glu Gly Lys Val Ile Thr Arg Gly
           595                 600                 605
   Leu Glu Ile Val Arg Arg Asp Trp Ser Glu Ile Ala Lys Glu Thr Gln
       610                 615                 620
   Ala Arg Val Leu Glu Thr Ile Leu Lys His Gly Asp Val Glu Glu Ala
   625                 630                 635                 640
   Val Arg Ile Val Lys Glu Val Ile Gln Lys Leu Ala Asn Tyr Glu Ile
                   645                 650                 655
   Pro Pro Glu Lys Leu Ala Ile Tyr Glu Gln Ile Thr Arg Pro Leu His
               660                 665                 670
   Glu Tyr Lys Ala Ile Gly Pro His Val Ala Val Ala Lys Lys Leu Ala
           675                 680                 685
   Ala Lys Gly Val Lys Ile Lys Pro Gly Met Val Ile Gly Tyr Ile Val
       690                 695                 700
   Leu Arg Gly Asp Gly Pro Ile Ser Asn Arg Ala Ile Leu Ala Glu Glu
   705                 710                 715                 720
   Tyr Asp Pro Lys Lys His Lys Tyr Asp Ala Glu Tyr Tyr Ile Glu Asn
                   725                 730                 735
   Gln Val Leu Pro Ala Val Leu Arg Ile Leu Glu Gly Phe Gly Tyr Arg
               740                 745                 750
   Lys Glu Asp Leu Arg Tyr Gln Lys Thr Arg Gln Val Gly Leu Thr Ser
           755                 760                 765
   Trp Leu Asn Ile Lys Lys Ser
       770                 775
<210> 2
   <211> 775
   <212> PRT
   <213> Pyrococcus furiosus
<400> 2
   Met Ile Leu Asp Val Asp Tyr Ile Thr Glu Glu Gly Lys Pro Val Ile
   1               5                   10                  15
   Arg Leu Phe Lys Lys Glu Asn Gly Lys Phe Lys Ile Glu His Asp Arg
               20                  25                  30
   Thr Phe Arg Pro Tyr Ile Tyr Ala Leu Leu Arg Asp Asp Ser Lys Ile
           35                  40                  45
   Glu Glu Val Lys Lys Ile Thr Gly Glu Arg His Gly Lys Ile Val Arg
       50                  55                  60
   Ile Val Asp Val Glu Lys Val Glu Lys Lys Phe Leu Gly Lys Pro Ile
   65                  70                  75                  80
   Thr Val Trp Lys Leu Tyr Leu Glu His Pro Gln Asp Val Pro Thr Ile
                   85                  90                  95
   Arg Glu Lys Val Arg Glu His Pro Ala Val Val Asp Ile Phe Glu Tyr
               100                 105                 110
   Asp Ile Pro Phe Ala Lys Arg Tyr Leu Ile Asp Lys Gly Leu Ile Pro
           115                 120                 125
   Met Glu Gly Glu Glu Glu Leu Lys Ile Leu Ala Phe Ala Ile Ala Thr
       130                 135                 140
   Leu Tyr His Glu Gly Glu Glu Phe Gly Lys Gly Pro Ile Ile Met Ile
   145                 150                 155                 160
   Ser Tyr Ala Asp Glu Asn Glu Ala Lys Val Ile Thr Trp Lys Asn Ile
                   165                 170                 175
   Asp Leu Pro Tyr Val Glu Val Val Ser Ser Glu Arg Glu Met Ile Lys
               180                 185                 190
   Arg Phe Leu Arg Ile Ile Arg Glu Lys Asp Pro Asp Ile Ile Val Thr
           195                 200                 205
   Tyr Asn Gly Asp Ser Phe Asp Phe Pro Tyr Leu Ala Lys Arg Ala Glu
       210                 215                 220
   Lys Leu Gly Ile Lys Leu Thr Ile Gly Arg Asp Gly Ser Glu Pro Lys
   225                 230                 235                 240
   Met Gln Arg Ile Gly Asp Met Thr Ala Val Glu Val Lys Gly Arg Ile
                   245                 250                 255
   His Phe Asp Leu Tyr His Val Ile Thr Arg Thr Ile Asn Leu Pro Thr
               260                 265                 270
   Tyr Thr Leu Glu Ala Val Tyr Glu Ala Ile Phe Gly Lys Pro Lys Glu
           275                 280                 285
   Lys Val Tyr Ala Asp Glu Ile Ala Lys Ala Trp Glu Ser Gly Glu Asn
       290                 295                 300
   Leu Glu Arg Val Ala Lys Tyr Ser Met Glu Asp Ala Lys Ala Thr Tyr
   305                 310                 315                 320
   Glu Leu Gly Lys Glu Phe Leu Pro Met Glu Ile Gln Leu Ser Arg Leu
                   325                 330                 335
   Val Gly Gln Pro Leu Trp Asp Val Ser Arg Ser Ser Thr Gly Asn Leu
               340                 345                 350
   Val Glu Trp Phe Leu Leu Arg Lys Ala Tyr Glu Arg Asn Glu Val Ala
   355 360 365
   Pro Asn Lys Pro Ser Glu Glu Glu Tyr Gln Arg Arg Leu Arg Glu Ser
   370 375 380
   Tyr Thr Gly Gly Phe Val Lys Glu Pro Glu Lys Gly Leu Trp Glu Asn
   385 390 395 400
   Ile Val Tyr Leu Asp Phe Arg Ala Ser Gly Ser Ser Ile Ile Ile Thr
   405 410 415
   His Asn Val Ser Pro Asp Thr Leu Asn Leu Glu Gly Cys Lys Asn Tyr
   420 425 430
   Asp Ile Ala Pro Gln Val Gly His Lys Phe Cys Lys Asp Ile Pro Gly
   435 440 445
   Phe Ile Pro Ser Leu Leu Gly His Leu Leu Glu Glu Arg Gln Lys Ile
   450 455 460
   Lys Thr Lys Met Lys Glu Thr Gln Asp Pro Ile Glu Lys Ile Leu Leu
   465 470 475 480
   Asp Tyr Arg Gln Lys Leu Ile Lys Leu Leu Ala Asn Ser Phe Tyr Gly
   485 490 495
   Tyr Tyr Gly Tyr Ala Lys Ala Arg Trp Tyr Cys Lys Glu Cys Ala Glu
   500 505 510
   Ser Val Thr Ala Trp Gly Arg Lys Tyr Ile Glu Leu Val Trp Lys Glu
   515 520 525
   Leu Glu Glu Lys Phe Gly Phe Lys Val Leu Tyr Ile Asp Thr Asp Gly
   530 535 540
   Leu Tyr Ala Thr Ile Pro Gly Gly Glu Ser Glu Glu Ile Lys Lys Lys
   545 550 555 560
   Ala Leu Glu Phe Val Lys Tyr Ile Asn Ser Lys Leu Pro Gly Leu Leu
   565 570 575
   Glu Leu Glu Tyr Glu Gly Phe Tyr Lys Arg Gly Phe Phe Val Thr Lys
   580 585 590
   Lys Arg Tyr Ala Val Ile Asp Glu Glu Gly Lys Val Ile Thr Arg Gly
   595 600 605
   Leu Glu Ile Val Arg Arg Asp Trp Ser Glu Ile Ala Lys Glu Thr Gln
       610                 615                 620
   Ala Arg Val Leu Glu Thr Ile Leu Lys His Gly Asp Val Glu Glu Ala
   625                 630                 635                 640
   Val Arg Ile Val Lys Glu Val Ile Gln Lys Leu Ala Asn Tyr Glu Ile
                   645                 650                 655
   Pro Pro Glu Lys Leu Ala Ile Tyr Glu Gln Ile Thr Arg Pro Leu His
               660                 665                 670
   Glu Tyr Lys Ala Ile Gly Pro His Val Ala Val Ala Lys Lys Leu Ala
           675                 680                 685
   Ala Lys Gly Val Lys Ile Lys Pro Gly Met Val Ile Gly Tyr Ile Val
       690                 695                 700
   Leu Arg Gly Asp Gly Pro Ile Ser Asn Arg Ala Ile Leu Ala Glu Glu
   705                 710                 715                 720
   Tyr Asp Pro Lys Lys His Lys Tyr Asp Ala Glu Tyr Tyr Ile Glu Asn
                   725                 730                 735
   Gln Val Leu Pro Ala Val Leu Arg Ile Leu Glu Gly Phe Gly Tyr Arg
               740                 745                 750
   Lys Glu Asp Leu Arg Tyr Gln Lys Thr Arg Gln Val Gly Leu Thr Ser
           755                 760                 765
   Trp Leu Asn Ile Lys Lys Ser
       770                 775
<210> 3
   <211> 2328
   <212> PRT
   <213> Pyrococcus furiosus
<400> 3
   Ala Thr Gly Ala Thr Thr Cys Thr Gly Gly Ala Thr Gly Thr Gly Gly
   1               5                   10                  15
   Ala Thr Thr Ala Cys Ala Thr Thr Ala Cys Cys Gly Ala Ala Gly Ala
               20                  25                  30
   Ala Gly Gly Cys Ala Ala Ala Cys Cys Gly Gly Thr Thr Ala Thr Thr
           35                  40                  45
   Cys Gly Cys Cys Thr Gly Thr Thr Cys Ala Ala Ala Ala Ala Ala Gly
       50                  55                  60
   Ala Ala Ala Ala Cys Gly Gly Cys Ala Ala Ala Thr Thr Cys Ala Ala
   65                  70                  75                  80
   Ala Ala Thr Cys Gly Ala Gly Cys Ala Cys Gly Ala Thr Cys Gly Thr
                   85                  90                  95
   Ala Cys Cys Thr Thr Thr Cys Gly Thr Cys Cys Gly Thr Ala Thr Ala
               100                 105                 110
   Thr Cys Thr Ala Thr Gly Cys Ala Cys Thr Gly Cys Thr Gly Cys Gly
           115                 120                 125
   Thr Gly Ala Thr Gly Ala Thr Ala Gly Cys Ala Ala Ala Ala Thr Cys
       130                 135                 140
   Gly Ala Ala Gly Ala Ala Gly Thr Gly Ala Ala Ala Ala Ala Ala Ala
   145                 150                 155                 160
   Thr Cys Ala Cys Gly Gly Gly Thr Gly Ala Ala Cys Gly Cys Cys Ala
                   165                 170                 175
   Thr Gly Gly Cys Ala Ala Ala Ala Thr Thr Gly Thr Thr Cys Gly Thr
               180                 185                 190
   Ala Thr Thr Gly Thr Thr Gly Ala Thr Gly Thr Cys Gly Ala Ala Ala
           195                 200                 205
   Ala Ala Gly Thr Cys Gly Ala Gly Ala Ala Ala Ala Ala Ala Thr Thr
       210                 215                 220
   Cys Cys Thr Gly Gly Gly Thr Ala Ala Ala Cys Cys Gly Ala Thr Thr
   225                 230                 235                 240
   Ala Cys Cys Gly Thr Gly Thr Gly Gly Ala Ala Ala Cys Thr Gly Thr
                   245                 250                 255
   Ala Thr Cys Thr Gly Gly Ala Ala Cys Ala Thr Cys Cys Gly Cys Ala
               260                 265                 270
   Gly Gly Ala Thr Gly Thr Gly Cys Cys Gly Ala Cys Cys Ala Thr Thr
           275                 280                 285
   Cys Gly Thr Gly Ala Ala Ala Ala Ala Gly Thr Thr Cys Gly Thr Gly
   290 295 300
   Ala Ala Cys Ala Thr Cys Cys Gly Gly Cys Ala Gly Thr Gly Gly Thr
   305 310 315 320
   Thr Gly Ala Thr Ala Thr Cys Thr Thr Thr Gly Ala Ala Thr Ala Thr
   325 330 335
   Gly Ala Thr Ala Thr Thr Cys Cys Gly Thr Thr Cys Gly Cys Cys Ala
   340 345 350
   Ala Ala Cys Gly Cys Thr Ala Cys Cys Thr Gly Ala Thr Thr Gly Ala
   355 360 365
   Thr Ala Ala Ala Gly Gly Thr Cys Thr Gly Ala Thr Thr Cys Cys Gly
   370 375 380
   Ala Thr Gly Gly Ala Ala Gly Gly Thr Gly Ala Ala Gly Ala Ala Gly
   385 390 395 400
   Ala Ala Cys Thr Gly Ala Ala Ala Ala Thr Thr Cys Thr Gly Gly Cys
   405 410 415
   Ala Thr Thr Thr Gly Cys Cys Ala Thr Thr Gly Cys Ala Ala Cys Cys
   420 425 430
   Cys Thr Gly Thr Ala Thr Cys Ala Thr Gly Ala Ala Gly Gly Cys Gly
   435 440 445
   Ala Ala Gly Ala Ala Thr Thr Thr Gly Gly Thr Ala Ala Ala Gly Gly
   450 455 460
   Thr Cys Cys Gly Ala Thr Thr Ala Thr Cys Ala Thr Gly Ala Thr Cys
   465 470 475 480
   Ala Gly Cys Thr Ala Thr Gly Cys Cys Gly Ala Thr Gly Ala Ala Ala
   485 490 495
   Ala Thr Gly Ala Gly Gly Cys Cys Ala Ala Ala Gly Thr Thr Ala Thr
   500 505 510
   Thr Ala Cys Cys Thr Gly Gly Ala Ala Ala Ala Ala Cys Ala Thr Cys
   515 520 525
   Gly Ala Thr Cys Thr Gly Cys Cys Gly Thr Ala Thr Gly Thr Thr Gly
   530 535 540
   Ala Ala Gly Thr Thr Gly Thr Thr Ala Gly Cys Ala Gly Cys Gly Ala
   545                 550                 555                 560
   Ala Cys Gly Thr Gly Ala Ala Ala Thr Gly Ala Thr Thr Ala Ala Ala
                   565                 570                 575
   Cys Gly Thr Thr Thr Thr Cys Thr Gly Cys Gly Cys Ala Thr Cys Ala
               580                 585                 590
   Thr Cys Cys Gly Cys Gly Ala Ala Ala Ala Ala Gly Ala Thr Cys Cys
           595                 600                 605
   Gly Gly Ala Thr Ala Thr Cys Ala Thr Thr Gly Thr Gly Ala Cys Cys
       610                 615                 620
   Thr Ala Thr Ala Ala Cys Gly Gly Cys Gly Ala Thr Ala Gly Cys Thr
   625                 630                 635                 640
   Thr Thr Gly Ala Thr Thr Thr Thr Cys Cys Gly Thr Ala Thr Cys Thr
                   645                 650                 655
   Gly Gly Cys Ala Ala Ala Ala Cys Gly Thr Gly Cys Ala Gly Ala Ala
               660                 665                 670
   Ala Ala Ala Cys Thr Gly Gly Gly Thr Ala Thr Thr Ala Ala Ala Cys
           675                 680                 685
   Thr Gly Ala Cys Cys Ala Thr Thr Gly Gly Thr Cys Gly Thr Gly Ala
       690                 695                 700
   Thr Gly Gly Thr Ala Gly Cys Gly Ala Ala Cys Cys Gly Ala Ala Ala
   705                 710                 715                 720
   Ala Thr Gly Cys Ala Gly Cys Gly Thr Ala Thr Thr Gly Gly Thr Gly
                   725                 730                 735
   Ala Thr Ala Thr Gly Ala Cys Cys Gly Cys Ala Gly Thr Thr Gly Ala
               740                 745                 750
   Ala Gly Thr Thr Ala Ala Ala Gly Gly Thr Cys Gly Cys Ala Thr Thr
           755                 760                 765
   Cys Ala Cys Thr Thr Thr Gly Ala Thr Cys Thr Gly Thr Ala Cys Cys
       770                 775                 780
   Ala Thr Gly Thr Thr Ala Thr Thr Ala Cys Cys Cys Gly Cys Ala Cys
   785                 790                 795                 800
   Cys Ala Thr Thr Ala Ala Thr Cys Thr Gly Cys Cys Gly Ala Cys Cys
   805 810 815
   Thr Ala Thr Ala Cys Cys Cys Thr Gly Gly Ala Ala Gly Cys Ala Gly
   820 825 830
   Thr Thr Thr Ala Thr Gly Ala Ala Gly Cys Ala Ala Thr Thr Thr Thr
   835 840 845
   Cys Gly Gly Cys Ala Ala Ala Cys Cys Gly Ala Ala Ala Gly Ala Ala
   850 855 860
   Ala Ala Ala Gly Thr Gly Thr Ala Thr Gly Cys Gly Gly Ala Thr Gly
   865 870 875 880
   Ala Ala Ala Thr Thr Gly Cys Ala Ala Ala Ala Gly Cys Ala Thr Gly
   885 890 895
   Gly Gly Ala Ala Ala Gly Cys Gly Gly Thr Gly Ala Ala Ala Ala Thr
   900 905 910
   Cys Thr Gly Gly Ala Ala Cys Gly Thr Gly Thr Thr Gly Cys Ala Ala
   915 920 925
   Ala Ala Thr Ala Thr Ala Gly Cys Ala Thr Gly Gly Ala Ala Gly Ala
   930 935 940
   Thr Gly Cys Ala Ala Ala Ala Gly Cys Cys Ala Cys Cys Thr Ala Thr
   945 950 955 960
   Gly Ala Ala Cys Thr Gly Gly Gly Thr Ala Ala Ala Gly Ala Ala Thr
   965 970 975
   Thr Thr Cys Thr Gly Cys Cys Gly Ala Thr Gly Gly Ala Ala Ala Thr
   980 985 990
   Thr Cys Ala Gly Cys Thr Gly Ala Gly Cys Cys Gly Thr Cys Thr Gly
   995 1000 1005
   Gly Thr Thr Gly Gly Thr Cys Ala Gly Cys Cys Gly Cys Thr Gly
   1010 1015 1020
   Thr Gly Gly Gly Ala Thr Gly Thr Thr Ala Gly Cys Cys Gly Thr
   1025 1030 1035
   Ala Gly Cys Ala Gly Cys Ala Cys Cys Gly Gly Thr Ala Ala Thr
   1040 1045 1050
   Cys Thr  Gly Gly Thr Thr Gly  Ala Ala Thr Gly Gly  Thr Thr Thr
       1055                 1060                 1065
   Cys Thr  Gly Cys Thr Gly Cys  Gly Thr Ala Ala Ala  Gly Cys Cys
       1070                 1075                 1080
   Thr Ala  Thr Gly Ala Ala Cys  Gly Thr Ala Ala Thr  Gly Ala Ala
       1085                 1090                 1095
   Gly Thr  Thr Gly Cys Ala Cys  Cys Gly Ala Ala Thr  Ala Ala Ala
       1100                 1105                 1110
   Cys Cys  Gly Ala Gly Cys Gly  Ala Ala Gly Ala Ala  Gly Ala Ala
       1115                 1120                 1125
   Thr Ala  Thr Cys Ala Gly Cys  Gly Thr Cys Gly Thr  Cys Thr Gly
       1130                 1135                 1140
   Cys Gly  Thr Gly Ala Ala Ala  Gly Cys Thr Ala Thr  Ala Cys Cys
       1145                 1150                 1155
   Gly Gly  Thr Gly Gly Thr Thr  Thr Thr Gly Thr Thr  Ala Ala Ala
       1160                 1165                 1170
   Gly Ala  Ala Cys Cys Gly Gly  Ala Ala Ala Ala Ala  Gly Gly Thr
       1175                 1180                 1185
   Cys Thr  Gly Thr Gly Gly Gly  Ala Gly Ala Ala Thr  Ala Thr Cys
       1190                 1195                 1200
   Gly Thr  Thr Thr Ala Thr Cys  Thr Gly Gly Ala Thr  Thr Thr Thr
       1205                 1210                 1215
   Cys Gly  Thr Gly Cys Ala Ala  Gly Cys Gly Gly Thr  Ala Gly Cys
       1220                 1225                 1230
   Ala Gly  Cys Ala Thr Thr Ala  Thr Thr Ala Thr Cys  Ala Cys Cys
       1235                 1240                 1245
   Cys Ala  Thr Ala Ala Thr Gly  Thr Thr Ala Gly Thr  Cys Cys Gly
       1250                 1255                 1260
   Gly Ala  Thr Ala Cys Cys Cys  Thr Gly Ala Ala Thr  Cys Thr Gly
       1265                 1270                 1275
   Gly Ala  Ala Gly Gly Cys Thr  Gly Thr Ala Ala Ala  Ala Ala Cys
   1280 1285 1290
   Thr Ala Thr Gly Ala Thr Ala Thr Thr Gly Cys Ala Cys Cys Gly
   1295 1300 1305
   Cys Ala Gly Gly Thr Thr Gly Gly Cys Cys Ala Cys Ala Ala Ala
   1310 1315 1320
   Thr Thr Cys Thr Gly Thr Ala Ala Ala Gly Ala Thr Ala Thr Thr
   1325 1330 1335
   Cys Cys Gly Gly Gly Thr Thr Thr Thr Ala Thr Thr Cys Cys Gly
   1340 1345 1350
   Ala Gly Cys Cys Thr Gly Cys Thr Gly Gly Gly Thr Cys Ala Thr
   1355 1360 1365
   Cys Thr Gly Cys Thr Gly Gly Ala Ala Gly Ala Ala Cys Gly Thr
   1370 1375 1380
   Cys Ala Ala Ala Ala Ala Ala Thr Cys Ala Ala Ala Ala Cys Cys
   1385 1390 1395
   Ala Ala Ala Ala Thr Gly Ala Ala Ala Gly Ala Ala Ala Cys Cys
   1400 1405 1410
   Cys Ala Gly Gly Ala Thr Cys Cys Gly Ala Thr Cys Gly Ala Ala
   1415 1420 1425
   Ala Ala Ala Ala Thr Cys Cys Thr Gly Cys Thr Gly Gly Ala Thr
   1430 1435 1440
   Thr Ala Thr Cys Gly Thr Cys Ala Gly Ala Ala Ala Cys Thr Gly
   1445 1450 1455
   Ala Thr Cys Ala Ala Ala Cys Thr Gly Cys Thr Gly Gly Cys Cys
   1460 1465 1470
   Ala Ala Thr Ala Gly Cys Thr Thr Cys Thr Ala Thr Gly Gly Thr
   1475 1480 1485
   Thr Ala Thr Thr Ala Thr Gly Gly Cys Thr Ala Thr Gly Cys Cys
   1490 1495 1500
   Ala Ala Ala Gly Cys Ala Cys Gly Cys Thr Gly Gly Thr Ala Thr
   1505 1510 1515
   Thr Gly  Thr Ala Ala Ala Gly  Ala Ala Thr Gly Thr  Gly Cys Ala
       1520                 1525                 1530
   Gly Ala  Ala Ala Gly Cys Gly  Thr Thr Ala Cys Cys  Gly Cys Ala
       1535                 1540                 1545
   Thr Gly  Gly Gly Gly Thr Cys  Gly Cys Ala Ala Ala  Thr Ala Thr
       1550                 1555                 1560
   Ala Thr  Cys Gly Ala Ala Cys  Thr Gly Gly Thr Thr  Thr Gly Gly
       1565                 1570                 1575
   Ala Ala  Ala Gly Ala Ala Cys  Thr Gly Gly Ala Ala  Gly Ala Gly
       1580                 1585                 1590
   Ala Ala  Ala Thr Thr Thr Gly  Gly Cys Thr Thr Cys  Ala Ala Ala
       1595                 1600                 1605
   Gly Thr  Gly Cys Thr Gly Thr  Ala Thr Ala Thr Thr  Gly Ala Thr
       1610                 1615                 1620
   Ala Cys  Cys Gly Ala Thr Gly  Gly Thr Cys Thr Gly  Thr Ala Thr
       1625                 1630                 1635
   Gly Cys  Ala Ala Cys Cys Ala  Thr Thr Cys Cys Gly  Gly Gly Thr
       1640                 1645                 1650
   Gly Gly  Thr Gly Ala Ala Ala  Gly Cys Gly Ala Ala  Gly Ala Gly
       1655                 1660                 1665
   Ala Thr  Thr Ala Ala Ala Ala  Ala Ala Ala Ala Ala  Gly Cys Cys
       1670                 1675                 1680
   Cys Thr  Gly Gly Ala Ala Thr  Thr Thr Gly Thr Gly  Ala Ala Ala
       1685                 1690                 1695
   Thr Ala  Cys Ala Thr Cys Ala  Ala Cys Ala Gly Cys  Ala Ala Ala
       1700                 1705                 1710
   Cys Thr  Gly Cys Cys Ala Gly  Gly Cys Cys Thr Gly  Cys Thr Gly
       1715                 1720                 1725
   Gly Ala  Ala Cys Thr Gly Gly  Ala Ala Thr Ala Thr  Gly Ala Ala
       1730                 1735                 1740
   Gly Gly  Thr Thr Thr Cys Thr  Ala Thr Ala Ala Ala  Cys Gly Cys
       1745                 1750                 1755
   Gly Gly Thr Thr Thr Cys Thr Thr Thr Gly Thr Gly Ala Cys Cys
   1760 1765 1770
   Ala Ala Ala Ala Ala Ala Cys Gly Cys Thr Ala Thr Gly Cys Ala
   1775 1780 1785
   Gly Thr Gly Ala Thr Thr Gly Ala Thr Gly Ala Ala Gly Ala Gly
   1790 1795 1800
   Gly Gly Thr Ala Ala Ala Gly Thr Gly Ala Thr Thr Ala Cys Cys
   1805 1810 1815
   Cys Gly Thr Gly Gly Thr Cys Thr Gly Gly Ala Ala Ala Thr Thr
   1820 1825 1830
   Gly Thr Gly Cys Gly Thr Cys Gly Thr Gly Ala Thr Thr Gly Gly
   1835 1840 1845
   Ala Gly Thr Gly Ala Ala Ala Thr Cys Gly Cys Cys Ala Ala Ala
   1850 1855 1860
   Gly Ala Ala Ala Cys Ala Cys Ala Gly Gly Cys Ala Cys Gly Thr
   1865 1870 1875
   Gly Thr Thr Cys Thr Gly Gly Ala Ala Ala Cys Ala Ala Thr Thr
   1880 1885 1890
   Cys Thr Gly Ala Ala Ala Cys Ala Thr Gly Gly Thr Gly Ala Thr
   1895 1900 1905
   Gly Thr Thr Gly Ala Ala Gly Ala Ala Gly Cys Cys Gly Thr Gly
   1910 1915 1920
   Cys Gly Thr Ala Thr Cys Gly Thr Thr Ala Ala Ala Gly Ala Ala
   1925 1930 1935
   Gly Thr Thr Ala Thr Cys Cys Ala Gly Ala Ala Ala Cys Thr Gly
   1940 1945 1950
   Gly Cys Cys Ala Ala Cys Thr Ala Thr Gly Ala Ala Ala Thr Thr
   1955 1960 1965
   Cys Cys Gly Cys Cys Thr Gly Ala Ala Ala Ala Ala Cys Thr Gly
   1970 1975 1980
   Gly Cys Ala Ala Thr Cys Thr Ala Thr Gly Ala Gly Cys Ala Gly
   1985 1990 1995
   Ala Thr  Thr Ala Cys Cys Cys  Gly Thr Cys Cys Gly  Cys Thr Gly
       2000                 2005                 2010
   Cys Ala  Thr Gly Ala Ala Thr  Ala Thr Ala Ala Ala  Gly Cys Ala
       2015                 2020                 2025
   Ala Thr  Thr Gly Gly Thr Cys  Cys Gly Cys Ala Thr  Gly Thr Thr
       2030                 2035                 2040
   Gly Cys  Cys Gly Thr Thr Gly  Cys Ala Ala Ala Ala  Ala Ala Ala
       2045                 2050                 2055
   Cys Thr  Gly Gly Cys Thr Gly  Cys Ala Ala Ala Ala  Gly Gly Cys
       2060                 2065                 2070
   Gly Thr  Thr Ala Ala Ala Ala  Thr Cys Ala Ala Ala  Cys Cys Gly
       2075                 2080                 2085
   Gly Gly  Thr Ala Thr Gly Gly  Thr Gly Ala Thr Thr  Gly Gly Thr
       2090                 2095                 2100
   Thr Ala  Thr Ala Thr Thr Gly  Thr Thr Cys Thr Gly  Cys Gly Thr
       2105                 2110                 2115
   Gly Gly  Thr Gly Ala Thr Gly  Gly Thr Cys Cys Gly  Ala Thr Thr
       2120                 2125                 2130
   Thr Cys  Ala Ala Ala Thr Cys  Gly Thr Gly Cys Ala  Ala Thr Thr
       2135                 2140                 2145
   Cys Thr  Gly Gly Cys Cys Gly  Ala Ala Gly Ala Ala  Thr Ala Cys
       2150                 2155                 2160
   Gly Ala  Thr Cys Cys Gly Ala  Ala Ala Ala Ala Ala  Cys Ala Cys
       2165                 2170                 2175
   Ala Ala  Ala Thr Ala Thr Gly  Ala Cys Gly Cys Cys  Gly Ala Gly
       2180                 2185                 2190
   Thr Ala  Thr Thr Ala Thr Ala  Thr Cys Gly Ala Ala  Ala Ala Thr
       2195                 2200                 2205
   Cys Ala  Gly Gly Thr Thr Cys  Thr Gly Cys Cys Thr  Gly Cys Ala
       2210                 2215                 2220
   Gly Thr  Thr Cys Thr Gly Cys  Gly Thr Ala Thr Thr  Cys Thr Gly
           2225                2230                2235
   Gly Ala  Ala Gly Gly Thr Thr  Thr Thr Gly Gly Thr  Thr Ala Thr
       2240                2245                2250
   Cys Gly  Cys Ala Ala Ala Gly  Ala Ala Gly Ala Thr  Cys Thr Gly
       2255                2260                2265
   Cys Gly  Thr Thr Ala Thr Cys  Ala Gly Ala Ala Ala  Ala Cys Cys
       2270                2275                2280
   Cys Gly  Thr Cys Ala Gly Gly  Thr Gly Gly Gly Thr  Cys Thr Gly
       2285                2290                2295
   Ala Cys  Cys Ala Gly Cys Thr  Gly Gly Cys Thr Gly  Ala Ala Thr
       2300                2305                2310
   Ala Thr  Cys Ala Ala Ala Ala  Ala Ala Ala Gly Cys  Thr Ala Ala
       2315                2320                2325